# EUROPEAN PATENT SPECIFICATION

(11) **EP 0 711 833 B1**
(45) Date of publication and mention of the grant of the patent: **03.08.2005**
(21) Application number: 95402335.4
(22) Date of filing: 19.10.1995
(51) Int. Cl.: C12N 15/12, C07K 14/47, C12N 15/11, C12Q 1/68, C12N 5/10, C07K 16/18

(54) **Survival motor neuron (SMN) gene: a gene for spinal muscular atrophy**
Motorneuronlebenserhaltungsgen: ein Gen für spinale Muskelatrophie
Gène de survie des neurones moteurs: gène de l'atrophie musculaire spinale

(30) Priority: 19.10.1994 EP 94402353
(43) Date of publication of application: 15.05.1996
(73) Proprietor: INSTITUT NATIONAL DE LA SANTE ET DE LA RECHERCHE MEDICALE (INSERM), 75654 Paris Cédex 13 (FR)
(72) Inventor: Melki, Judith, 75015 Paris (FR); Munnich, Arnold, 75006 Paris (FR)
(74) Representative: Desaix, Anne

(56) References cited:
- WO-A-92/00386
- WO-A-95/33852
- SCIENCE, vol. 264, 3 June 1994, pages 1474-1477, XP002022184 J. MELKI ET AL: "De novo and inherited deletions of the 5q13 region in spinal muscular atrophies"
- THE AMERICAN JOURNAL OF HUMAN GENETICS, vol. 54, no. 4, April 1994, pages 687-594, XP000615587 O. CLERMONT ET AL: "Use of genetic and physical mapping to locate the spinal muscular atrophy locus between two new highly polymorhic DNA markers"
- SEMINARS IN NEUROLOGY, vol. 13, no. 3, September 1993, pages 276-282, XP002022185 P. KLEYN AND T. GILLIAM: "Progress toward cloning of the gene responsible for childhood spinal muscular atrophy"
- THE AMERICAN JOURNAL OF HUMAN GENETICS, vol. 55, no. 3, September 1994, page A31 XP002022186 C. DIDONATO ET AL: "Identification of strong allele association and candidate cDNAs for the spinal muscular atrophy gene"
- THE AMERICAN JOURNAL OF HUMAN GENETICS, vol. 55, no. 6, December 1994, pages 1218-1229, XP000615820 C. DIDENATO ET AL: "Association between AgI-CA alleles and severity of autosomal recessive proximal spinal muscular atrophy"
- THE AMERICAN JOURNAL OF HUMAN GENETICS, vol. 55, no. 3, September 1994, page A2691 XP002022187 B. ROSS ET AL: "Isolation of SMA candidate genes from a YAC contig by direct selection of cDNA from normalized cDNA libraries"
- TRENDS IN BIOTECHNOLOGY., vol. 5, no. 4, 1987, CAMBRIDGE GB, pages 107-111, XP002022188 D. KINGSBURY: "DNA probes in the diagnosis of genetic and infectious diseases"
- THE AMERICAN JOURNAL OF HUMAN GENETICS, vol. 55, no. 3, September 1994, page A31 XP002022189 T. CARTER ET AL: "Identification of candidate genes in the spinal muscular atrophy gene region"
- HUMAN PATHOLOGY, vol. 17, 1986, pages 656-672, XP000615582 B. BANKER: "Arthrogryposis multiplex congenita"
- CELL, vol. 80, 13 January 1995, pages 155-165, XP002022190 S LEFEBVRE ET AL: "Identification and characterization of a spinal muscular atrophy-determining gene"
- Pharmacia Biotech Catalogue, 1991, pages 96-100
- CORCIA P. ET AL: 'Abnormal SMN1 gene copy number is a susceptibility factor for amyotrophic lateral sclerosis' ANN.NEUROL. vol. 51, no. 2, February 2002, pages 243 - 246
- BÜRGLEN L. ET AL: 'Survival motor neuron gene deletion in the arthrogryposis multiplex congenita-spinal muscular atrophy association' J.CLIN.INVEST. vol. 98, no. 5, September 1996, pages 1130 - 1132

### SEQUENCE LISTING

(1) GENERAL INFORMATION:
   (i) APPLICANT:
      (A) NAME: INSTITUT NATIONAL DE LA SANTE ET DE LA RECHERCHE MEDICALE
      (B) STREET: 101 Rue de Tolbiac
      (C) CITY: PARIS
      (E) COUNTRY: FRANCE ,
      (F) POSTAL CODE (ZIP): 75654
   (ii) TITLE OF INVENTION: SURVIVAL MOTOR NEURON (SMN) GENE: A GENE FOR SPINAL MUSCULAR ATROPHY
   (iii) NUMBER OF SEQUENCES: 66
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 95402335.4
(2) INFORMATION FOR SEQ ID NO: 1:
   (i) SEQUENCE CHARACTERISTICS:
      (A) LENGTH: 347 base pairs
      (B) TYPE: nucleic acid
      (C) STRANDEDNESS: double
      (D) TOPOLOGY: linear
         (ii) MOLECULE TYPE: DNA (genomic)
         (iii) HYPOTHETICAL: NO
         (iv) ANTI-SENSE: NO
         (xi) SEQUENCE DESCRIPTION: SEQ ID NO: 1:
   AATTTTTAAA TTTTTTGTAG AGACAGGGTC TCATTATGTT GCCCAGGGTG GTGTCAAGCT     60
   CCAGGTCTCA AGTGATCCCC CTACCTCCGC CTCCCAAAGT TGTGGGATTG TAGGCATGAG    120
   CCACTGCAAG AAAACCTTAA CTGCAGCCTA ATAATTGTTT TCTTTGGGAT AACTTTTAAA    180
   GTACATTAAA AGACTATCAA CTTAATTTCT GATCATATTT TGTTGAATAA AATAAGTAAA    240
   ATGTCTTGTG AACAAAATGC TTTTTAACAT CCATATAAAG CTATCTATAT ATAGCTATCT 300
   ATGTCTATAT AGCTATTTTT TTTAACTTCC TTTTATTTTC CTTACAG 347
(2) INFORMATION FOR SEQ ID NO: 2:
   (i) SEQUENCE CHARACTERISTICS:
      (A) LENGTH: 444 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 2:
   GTAAGTCTGC CAGCATTATG AAAGTGAATC TTACTTTTGT AAAACTTTAT GGTTTGTGGA 60
   AAACAAATGT TTTTGAACAG TTAAAAAGTT CAGATGTTAA AAAGTTGAAA GGTTAATGTA 120
   AAACAATCAA TATTAAAGAA TTTTGATGCC AAAACTATTA GATAAAAGGT TAATCTACAT 180
   CCCTACTAGA ATTCTCATAC TTAACTGGTT GGTTATGTGG AAGAAACATA CTTTCACAAT 240
   AAAGAGCTTT AGGATATGAT GCCATTTTAT ATCACTAGTA GGCAGACCAG CAGACTTTTT 300
   TTTATTGTGA TATGGGATAA CCTAGGCATA CTGCACTGTA CACTCTGACA TATGAAGTGC 360
   TCTAGTCAAG TTTAACTGGT GTCCACAGAG GACATGGTTT AACTGGAATT CGTCAAGCCT 420
   CTGGTTCTAA TTTCTCATTT GCAG 444
(2) INFORMATION FOR SEQ ID NO: 3:
   (i) SEQUENCE CHARACTERISTICS:
      (A) LENGTH: 347 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 3:
   AATTTTTAAA TTTTTTGTAG AGACAGGGTC TCATTATGTT GCCCAGGGTG GTGTCAAGCT 60
   CCAGGTCTCA AGTGATCCCC CTACCTCCGC CTCCCAAAGT TGTGGGATTG TAGGCATGAG 120
   CCACTGCAAG AAAACCTTAA CTGCAGCCTA ATAATTGTTT TCTTTGGGAT AACTTTTAAA 180
   GTACATTAAA AGACTATCAA CTTAATTTCT GATCATATTT TGTTGAATAA AATAAGTAAA 240
   ATGTCTTGTG AACAAAATGC TTTTTAACAT CCATATAAAG CTATCTATAT ATAGCTATCT 300
   ATATCTATAT AGCTATTTTT TTTAACTTCC TTTTATTTTC CTTACAG 347
(2) INFORMATION FOR SEQ ID NO: 4:
   (i) SEQUENCE CHARACTERISTICS:
      (A) LENGTH: 444 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 4:
   GTAAGTCTGC CAGCATTATG AAAGTGAATC TTACTTTTGT AAAACTTTAT GGTTTGTGGA 60
   AAACAAATGT TTTTGAACAG TTAAAAAGTT CAGATGTTAG AAAGTTGAAA GGTTAATGTA 120
   AAACAATCAA TATTAAAGAA TTTTGATGCC AAAACTATTA GATAAAAGGT TAATCTACAT 180
   CCCTACTAGA ATTCTCATAC TTAACTGGTT GGTTGTGTGG AAGAAACATA CTTTCACAAT 240
   AAAGAGCTTT AGGATATGAT GCCATTTTAT ATCACTAGTA GGCAGACCAG CAGACTTTTT 300
   TTTATTGTGA TATGGGATAA CCTAGGCATA CTGCACTGTA CACTCTGACA TATGAAGTGC 360
   TCTAGTCAAG TTTAACTGGT GTCCACAGAG GACATGGTTT AACTGGAATT CGTCAAGCCT 420
   CTGGTTCTAA TTTCTCATTT GCAG 444
(2) INFORMATION FOR SEQ ID NO: 5:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (xi) SEQUENCE DESCRIPTION: SEQ ID NO: 5:
   AGACTATCAA CTTAATTTCT GATCA 25
(2) INFORMATION FOR SEQ ID NO: 6:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic acid"
   (iii) HYPOTHETICAL: NO
   (xi) SEQUENCE DESCRIPTION: SEQ ID NO: 6:
   TAAGGAATGT GAGCACCTTC CTTC 24
(2) INFORMATION FOR SEQ ID NO: 7:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 7:
   GTAATAACCA AATGCAATGT GAA 23
(2) INFORMATION FOR SEQ ID NO: 8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 8:
   CTACAACACC CTTCTCACAG 20
(2) INFORMATION FOR SEQ ID NO: 9:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 9:
   AGACTATCAA CTTAATTTCT GATCA 25
(2) INFORMATION FOR SEQ ID NO: 10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 10:
   TAAGGAATGT GAGCACCTTC CTTC 24
(2) INFORMATION FOR SEQ ID NO: 11:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 11:
   AGGGCGAGGC TCTGTCTCA 19
(2) INFORMATION FOR SEQ ID NO: 12:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 12:
   CGGGAGGACC GCTTGTAGT 19
(2) INFORMATION FOR SEQ ID NO: 13:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 13:
   GCCGGAAGTC GTCACTCTT 19
(2) INFORMATION FOR SEQ ID NO: 14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 14:
   GGGTGCTGAG AGCGCTAATA 20
(2) INFORMATION FOR SEQ ID NO: 15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 15:
   TGTGTGGATT AAGATGACTC 20
(2) INFORMATION FOR SEQ ID NO: 16:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 16:
   CACTTTATCG TATGTTATC 19
(2) INFORMATION FOR SEQ ID NO: 17:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 17:
   CTGTGCACCA CCCTGTAACA TG 22
(2) INFORMATION FOR SEQ ID NO: 18:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 18:
   AAGGACTAAT GAGACATCC 19
(2) INFORMATION FOR SEQ ID NO: 19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 19:
   CGAGATGATA GTTTGCCCTC 20
(2) INFORMATION FOR SEQ ID NO: 20:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 20:
   AGCTACTTCA CAGATTGGGG AAAG 24
(2) INFORMATION FOR SEQ ID NO: 21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 21:
   CTCATCTAGT CTCTGCTTCC 20
(2) INFORMATION FOR SEQ ID NO: 22:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 22:
   TGGATATGGA AATAGAGAGG GAGC 24
(2) INFORMATION FOR SEQ ID NO: 23:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 23:
   CACCCTTATA ACAAAAACCT GC 22
(2) INFORMATION FOR SEQ ID NO: 24:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 24:
   GAGAAAGGAG TTCCATGGAG CAG 23
(2) INFORMATION FOR SEQ ID NO: 25:
   (i) SEQUENCE CHARACTERISTICS:
      (A) LENGTH: 20 base pairs
      (B) TYPE: nucleic acid
      (C) STRANDEDNESS: single
   (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 25:
   GAGAGGTTAA ATGTCCCGAC 20
(2) INFORMATION FOR SEQ ID NO: 26:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 26:
   GTGAGAACTC CAGGTCTCCT GG 22
(2) INFORMATION FOR SEQ ID NO: 27:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 27:
   TGAGTCTGTT TGACTTCAGG 20
(2) INFORMATION FOR SEQ ID NO: 28:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 28:
   GAAGGAAATG GAGGCAGCCA GC 22
(2) INFORMATION FOR SEQ ID NO: 29:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 29:
   TTTCTACCCA TTAGAATCTG G                                21
(2) INFORMATION FOR SEQ ID NO: 30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 30:
   CCCCACTTAC TATCATGCTG GCTG                             24
(2) INFORMATION FOR SEQ ID NO: 31:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 31:
   CCAGACTTTA CTTTTTGTTT ACTG 24
(2) INFORMATION FOR SEQ ID NO: 32:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 32:
   ATAGCCACTC ATGTACCATG A 21
(2) INFORMATION FOR SEQ ID NO: 33:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 33:
   AAGAGTAATT TAAGCCTCAG ACAG 24
(2) INFORMATION FOR SEQ ID NO: 34:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 34:
   CTCCCATATG TCCAGATTCT CTTG 24
(2) INFORMATION FOR SEQ ID NO: 35:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 35:
   AGACTATCAA CTTAATTTCT GATCA 25
(2) INFORMATION FOR SEQ ID NO: 36:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 36:
   TAAGGAATGT GAGCACCTTC CTTC 24
(2) INFORMATION FOR SEQ ID NO: 37:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 37:
   AGACTATCAA CTTAATTTCT GATCA 25
(2) INFORMATION FOR SEQ ID NO: 38:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 38:
   GTAAGATTCA CTTTCATAAT GCTG 24
(2) INFORMATION FOR SEQ ID NO: 39:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 39:
   CTTTATGGTT TGTGGAAAAC A 21
(2) INFORMATION FOR SEQ ID NO: 40:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 40:
   GGCATCATAT CCTAAAGCTC 20
(2) INFORMATION FOR SEQ ID NO: 41:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 41:
   GTAATAACCA AATGCAATGT GAA 23
(2) INFORMATION FOR SEQ ID NO: 42:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 42:
   CTACAACACC CTTCTCACAG 20
(2) INFORMATION FOR SEQ ID NO: 43:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 43:
   GGTGTCCACA GAGGACATGG 20
(2) INFORMATION FOR SEQ ID NO: 44:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 44:
   AAGAGTTAAC CCATTCCAGC TTCC 24
(2) INFORMATION FOR SEQ ID NO: 45:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 45:
   Gly Gly Val Pro Glu Gln Glu Asp Ser Val Leu Phe Arg Arg Gly Thr
   1 5 10 15
(2) INFORMATION FOR SEQ ID NO: 46:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 46:
   Ser Arg Ser Pro Gly Asn Lys Ser Asp Asn Ile Lys Pro Lys
   1 5 10
(2) INFORMATION FOR SEQ ID NO: 47:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO: 47:
   Phe Arg Gln Asn Gln Lys Glu Gly Arg Cys Ser His Ser Leu Asn
   1               5                   10                  15
(2) INFORMATION FOR SEQ ID NO: 48:
   (i) SEQUENCE CHARACTERISTICS:
      (A) LENGTH: 294 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (xi) SEQUENCE DESCRIPTION: SEQ ID NO: 48:
   Met Ala Met Ser Ser Gly Gly Ser Gly Gly Gly Val Pro Glu Gln Glu
   1 5 10 15
   Asp Ser Val Leu Phe Arg Arg Gly Thr Gly Gln Ser Asp Asp Ser Asp
   20 25 30
   Ile Trp Asp Asp Thr Ala Leu Ile Lys Ala Tyr Asp Lys Ala Val Ala
   35 40 45
   Ser Phe Lys His Ala Leu Lys Asn Gly Asp Ile Cys Glu Thr Ser Gly
   50 55 60
   Lys Pro Lys Thr Thr Pro Lys Arg Lys Pro Ala Lys Lys Asn Lys Ser
   65 70 75 80
   Gln Lys Lys Asn Thr Ala Ala Ser Leu Gln Gln Trp Lys Val Gly Asp
   85 90 95
   Lys Cys Ser Ala Ile Trp Ser Glu Asp Gly Cys Ile Tyr Pro Ala Thr
   100 105 110
   Ile Ala Ser Ile Asp Phe Lys Arg Glu Thr Cys Val Val Val Tyr Thr
   115 120 125
   Gly Tyr Gly Asn Arg Glu Glu Gln Asn Leu Ser Asp Leu Leu Ser Pro
   130 135 140
   Ile Cys Glu Val Ala Asn Asn Ile Glu Gln Asn Ala Gln Glu Asn Glu
   145 150 155 160
   Asn Glu Ser Gln Val Ser Thr Asp Glu Ser Glu Asn Ser Arg Ser Pro
   165 170 175
   Gly Asn Lys Ser Asp Asn Ile Lys Pro Lys Ser Ala Pro Trp Asn Ser
   180 185 190
   Phe Leu Pro Pro Pro Pro Pro Met Pro Gly Pro Arg Leu Gly Pro Gly
   195 200 205
   Lys Pro Gly Leu Lys Phe Asn Gly Pro Pro Pro Pro Pro Pro Pro Pro
   210 215 220
   Pro Pro His Leu Leu Ser Cys Trp Leu Pro Pro Phe Pro Ser Gly Pro
   225 230 235 240
   Pro Ile Ile Pro Pro Pro Pro Pro Ile Cys Pro Asp Ser Leu Asp Asp
   245 250 255
   Ala Asp Ala Leu Gly Ser Met Leu Ile Ser Trp Tyr Met Ser Gly Tyr
   260 265 270
   His Thr Gly Tyr Tyr Met Gly Phe Arg Gln Asn Gln Lys Glu Gly Arg
   275 280 285
   Cys Ser His Ser Leu Asn
   290
(2) INFORMATION FOR SEQ ID NO: 49:
   (i) SEQUENCE CHARACTERISTICS:
      (A) LENGTH: 1582 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 49:
   CGGGGCCCCA CGCTGCGCAC CCGCGGGTTT GCTATGGCGA TGAGCAGCGG CGGCAGTGGT    60
   GGCGGCGTCC CGGAGCAGGA GGATTCCGTG CTGTTCCGGC GCGGCACAGG CCAGAGCGAT   120
   GATTCTGACA TTTGGGATGA TACAGCACTG ATAAAAGCAT ATGATAAAGC TGTGGCTTCA   180
   TTTAAGCATG CTCTAAAGAA TGGTGACATT TGTGAAACTT CGGGTAAACC AAAAACCACA   240
   CCTAAAAGAA AACCTGCTAA GAAGAATAAA AGCCAAAAGA AGAATACTGC AGCTTCCTTA   300
   CAACAGTGGA AAGTTGGGGA CAAATGTTCT GCCATTTGGT CAGAAGACGG TTGCATTTAC   360
   CCAGCTACCA TTGCTTCAAT TGATTTTAAG AGAGAAACCT GTGTTGTGGT TTACACTGGA   420
   TATGGAAATA GAGAGGAGCA AAATCTGTCC GATCTACTTT CCCCAATCTG TGAAGTAGCT   480
   AATAATATAG AACAGAATGC TCAAGAGAAT GAAAATGAAA GCCAAGTTTC AACAGATGAA   540
   AGTGAGAACT CCAGGTCTCC TGGAAATAAA TCAGATAACA TCAAGCCCAA ATCTGCTCCA   600
   TGGAACCCCT TTCTCCCTCC ACCACCCCCC ATGCCAGGGC CAAGACTGGG ACCAGGAAAG   660
   CCAGGTCTAA AATTCAATGG CCCACCACCG CCACCGCCAC CACCACCACC CCACTTACTA 720
   TCATGCTGGC TGCCTCCATT TCCTTCTGGA CCACCAATAA TTCCCCCACC ACCTCCCATA 780
   TGTCCAGATT CTCTTGATGA TGCTGATGCT TTGGGAAGTA TGTTAATTTC ATGGTACATG 840
   AGTGGCTATC ATACTGGCTA TTATATGGGT TTTAGACAAA ATCAAAAAGA AGGAAGGTGC 900
   TCACATTCCT TAAATTAAGG AGAAATGCTG GCATAGAGCA GCACTAAATG ACACCACTAA 960
   AGAAACGATC AGACAGATCT GGAATGTGAA GCGTTATAGA AGATAACTGG CCTCATTTCT 1020
   TCAAAATATC AAGTGTTGGG AAAGAAAAAA GGAAGTGGAA TGGGTAACTC TTCTTGATTA 1080
   AAAGTTATGT AATAACCAAA TGCAATGTGA AATATTTTAC TGGACTCTTT TGAAAAACCA 1140
   TCTGTAAAAG ACTGAGGTGG GGGTGGGAGG CCAGCACGGT GGTGAGGCAG TTGAGAAAAT 1200
   TTGAATGTGG ATTAGATTTT GAATGATATT GGATAATTAT TGGTAATTTT ATGGCCTGTG 1260
   AGAAGGGTGT TGTAGTTTAT AAAAGACTGT CTTAATTTGC ATACTTAAGC ATTTAGGAAT 1320
   GAAGTGTTAG AGTGTCTTAA AATGTTTCAA ATGGTTTAAC AAAATGTATG TGAGGCGTAT 1380
   GTGGCAAAAT GTTACAGAAT CTAACTGGTG GACATGGCTG TTCATTGTAC TGTTTTTTTC 1440
   TATCTTCTAT ATGTTTAAAA GTATATAATA AAAATATTTA ATTTTTTTTT AAAAAAAAAA 1500
   AAAAAAAAAA AAAAAAAAAA AAAAAAAAAA AAAAAAAAAA AAAAAAAAAA AAAAAAAAAA 1560
   AAAAAAAAAA AAAAAAAAAA AA 1582
(2) INFORMATION FOR SEQ ID NO: 50:
   (i) SEQUENCE CHARACTERISTICS:
      (A) LENGTH: 1408 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 50:
   AATTTTTAAA TTTTTTGTAG AGACAGGGTC TCATTATGTT GCCCAGGGTG GTGTCAAGCT 60
   CCAGGTCTCA AGTGATCCCC CTACCTCCGC CTCCCAAAGT TGTGGGATTG TAGGCATGAG 120
   CCACTGCAAG AAAACCTTAA CTGCAGCCTA ATAATTGTTT TCTTTGGGAT AACTTTTAAA 180
   GTACATTAAA AGACTATCAA CTTAATTTCT GATCATATTT TGTTGAATAA AATAAGTAAA     240
   ATGTCTTGTG AACAAAATGC TTTTTAACAT CCATATAAAG CTATCTATAT ATAGCTATCT     300
   ATATCTATAT AGCTATTTTT TTTAACTTCC TTTTATTTTC CTTACAGGGT TTTAGACAAA     360
   ATCAAAAAGA AGGAAGGTGC TCACATTCCT TAAATTAAGG AGTAAGTCTG CCAGCATTAT     420
   GAAAGTGAAT CTTACTTTTG TAAAACTTTA TGGTTTGTGG AAAACAAATG TTTTTGAACA     480
   GTTAAAAAGT TCAGATGTTA GAAAGTTGAA AGGTTAATGT AAAACAATCA ATATTAAAGA     540
   ATTTTGATGC CAAAACTATT AGATAAAAGG TTAATCTACA TCCCTACTAG AATTCTCATA     600
   CTTAACTGGT TGGTTGTGTG GAAGAAACAT ACTTTCACAA TAAAGAGCTT TAGGATATGA     660
   TGCCATTTTA TATCACTAGT AGGCAGACCA GCAGACTTTT TTTTATTGTG ATATGGGATA     720
   ACCTAGGCAT ACTGCACTGT ACACTCTGAC ATATGAAGTG CTCTAGTCAA GTTTAACTGG     780
   TGTCCACAGA GGACATGGTT TAACTGGAAT TCGTCAAGCC TCTGGTTCTA ATTTCTCATT     840
   TGCAGGAAAT GCTGGCATAG AGCAGCACTA AATGACACCA CTAAAGAAAC GATCAGACAG     900
   ATCTGGAATG TGAAGCGTTA TAGAAGATAA CTGGCCTCAT TTCTTCAAAA TATCAAGTGT     960
   TGGGAAAGAA AAAAGGAAGT GGAATGGGTA ACTCTTCTTG ATTAAAAGTT ATGTAATAAC    1020
   CAAATGCAAT GTGAAATATT TTACTGGACT CTTTTGAAAA ACCATCTGTA AAAGACTGAG    1080
   GTGGGGGTGG GAGGCCAGCA CGGTGGTGAG GCAGTTGAGA AAATTTGAAT GTGGATTAGA    1140
   TTTTGAATGA TATTGGATAA TTATTGGTAA TTTTATGGCC TGTGAGAAGG GTGTTGTAGT    1200
   TTATAAAAGA CTGTCTTAAT TTGCATACTT AAGCATTTAG GAATGAAGTG TTAGAGTGTC    1260
   TTAAAATGTT TCAAATGGTT TAACAAAATG TATGTGAGGC GTATGTGGCA AAATGTTACA    1320
   GAATCTAACT GGTGGACATG GCTGTTCATT GTACTGTTTT TTTCTATCTT CTATATGTTT    1380
   AAAAGTATAT AATAAAAATA TTTAATTT                                       1408
(2) INFORMATION FOR SEQ ID NO: 51:
   (i) SEQUENCE CHARACTERISTICS:
      (A) LENGTH: 1582 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 51:
   CGGGGCCCCA CGCTGCGCAT CCGCGGGTTT GCTATGGCGA TGAGCAGCGG CGGCAGTGGT     60
   GGCGGCGTCC CGGAGCAGGA GGATTCCGTG CTGTTCCGGC GCGGCACAGG CCAGAGCGAT    120
   GATTCTGACA TTTGGGATGA TACAGCACTG ATAAAAGCAT ATGATAAAGC TGTGGCTTCA    180
   TTTAAGCATG CTCTAAAGAA TGGTGACATT TGTGAAACTT CGGGTAAACC AAAAACCACA    240
   CCTAAAAGAA AACCTGCTAA GAAGAATAAA AGCCAAAAGA AGAATACTGC AGCTTCCTTA    300
   CAACAGTGGA AAGTTGGGGA CAAATGTTCT GCCATTTGGT CAGAAGACGG TTGCATTTAC    360
   CCAGCTACCA TTGCTTCAAT TGATTTTAAG AGAGAAACCT GTGTTGTGGT TTACACTGGA    420
   TATGGAAATA GAGAGGAGCA AAATCTGTCC GATCTACTTT CCCCAATCTG TGAAGTAGCT    480
   AATAATATAG AACAGAATGC TCAAGAGAAT GAAAATGAAA GCCAAGTTTC AACAGATGAA    540
   AGTGAGAACT CCAGGTCTCC TGGAAATAAA TCAGATAACA TCAAGCCCAA ATCTGCTCCA    600
   TGGAACTCTT TTCTCCCTCC ACCACCCCCC ATGCCAGGGC CAAGACTGGG ACCAGGAAAG    660
   CCAGGTCTAA AATTCAATGG CCCACCACCG CCACCGCCAC CACCACCACC CCACTTACTA    720
   TCATGCTGGC TGCCTCCATT TCCTTCTGGA CCACCAATAA TTCCCCCACC ACCTCCCATA    780
   TGTCCAGATT CTCTTGATGA TGCTGATGCT TTGGGAAGTA TGTTAATTTC ATGGTACATG    840
   AGTGGCTATC ATACTGGCTA TTATATGGGT TTCAGACAAA ATCAAAAAGA AGGAAGGTGC    900
   TCACATTCCT TAAATTAAGG AGAAATGCTG GCATAGAGCA GCACTAAATG ACACCACTAA    960
   AGAAACGATC AGACAGATCT GGAATGTGAA GCGTTATAGA AGATAACTGG CCTCATTTCT   1020
   TCAAAATATC AAGTGTTGGG AAAGAAAAAA GGAAGTGGAA TGGGTAACTC TTCTTGATTA   1080
   AAAGTTATGT AATAACCAAA TGCAATGTGA AATATTTTAC TGGACTCTTT TGAAAAACCA   1140
   TCTGTAAAAG ACTGGGGTGG GGGTGGGAGG CCAGCACGGT GGTGAGGCAG TTGAGAAAAT   1200
   TTGAATGTGG ATTAGATTTT GAATGATATT GGATAATTAT TGGTAATTTT ATGGCCTGTG   1260
   AGAAGGGTGT TGTAGTTTAT AAAAGACTGT CTTAATTTGC ATACTTAAGC ATTTAGGAAT   1320
   GAAGTGTTAG AGTGTCTTAA AATGTTTCAA ATGGTTTAAC AAAATGTATG TGAGGCGTAT   1380
   GTGGCAAAAT GTTACAGAAT CTAACTGGTG GACATGGCTG TTCATTGTAC TGTTTTTTTC   1440
   TATCTTCTAT ATGTTTAAAA GTATATAATA AAAATATTTA ATTTTTTTTT AAAAAAAAAA   1500
   AAAAAAAAAA AAAAAAAAAA AAAAAAAAAA AAAAAAAAAA AAAAAAAAAA AAAAAAAAAA   1560
   AAAAAAAAAA AAAAAAAAAA AA                                            1582
(2) INFORMATION FOR SEQ ID NO: 52:
   (i) SEQUENCE CHARACTERISTICS:
      (A) LENGTH: 1408 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 52:
   AATTTTTAAA TTTTTTGTAG AGACAGGGTC TCATTATGTT GCCCAGGGTG GTGTCAAGCT     60
   CCAGGTCTCA AGTGATCCCC CTACCTCCGC CTCCCAAAGT TGTGGGATTG TAGGCATGAG    120
   CCACTGCAAG AAAACCTTAA CTGCAGCCTA ATAATTGTTT TCTTTGGGAT AACTTTTAAA    180
   GTACATTAAA AGACTATCAA CTTAATTTCT GATCATATTT TGTTGAATAA AATAAGTAAA    240
   ATGTCTTGTG AACAAAATGC TTTTTAACAT CCATATAAAG CTATCTATAT ATAGCTATCT    300
   ATGTCTATAT AGCTATTTTT TTTAACTTCC TTTTATTTTC CTTACAGGGT TTCAGACAAA    360
   ATCAAAAAGA AGGAAGGTGC TCACATTCCT TAAATTAAGG AGTAAGTCTG CCAGCATTAT    420
   GAAAGTGAAT CTTACTTTTG TAAAACTTTA TGGTTTGTGG AAAACAAATG TTTTTGAACA    480
   GTTAAAAAGT TCAGATGTTA AAAAGTTGAA AGGTTAATGT AAAACAATCA ATATTAAAGA    540
   ATTTTGATGC CAAAACTATT AGATAAAAGG TTAATCTACA TCCCTACTAG AATTCTCATA    600
   CTTAACTGGT TGGTTATGTG GAAGAAACAT ACTTTCACAA TAAAGAGCTT TAGGATATGA    660
   TGCCATTTTA TATCACTAGT AGGCAGACCA GCAGACTTTT TTTTATTGTG ATATGGGATA    720
   ACCTAGGCAT ACTGCACTGT ACACTCTGAC ATATGAAGTG CTCTAGTCAA GTTTAACTGG    780
   TGTCCACAGA GGACATGGTT TAACTGGAAT TCGTCAAGCC TCTGGTTCTA ATTTCTCATT    840
   TGCAGGAAAT GCTGGCATAG AGCAGCACTA AATGACACCA CTAAAGAAAC GATCAGACAG    900
   ATCTGGAATG TGAAGCGTTA TAGAAGATAA CTGGCCTCAT TTCTTCAAAA TATCAAGTGT    960
   TGGGAAAGAA AAAAGGAAGT GGAATGGGTA ACTCTTCTTG ATTAAAAGTT ATGTAATAAC   1020
   CAAATGCAAT GTGAAATATT TTACTGGACT CTTTTGAAAA ACCATCTGTA AAAGACTGGG   1080
   GTGGGGGTGG GAGGCCAGCA CGGTGGTGAG GCAGTTGAGA AAATTTGAAT GTGGATTAGA   1140
   TTTTGAATGA TATTGGATAA TTATTGGTAA TTTTATGGCC TGTGAGAAGG GTGTTGTAGT   1200
   TTATAAAAGA CTGTCTTAAT TTGCATACTT AAGCATTTAG GAATGAAGTG TTAGAGTGTC   1260
   TTAAAATGTT TCAAATGGTT TAACAAAATG TATGTGAGGC GTATGTGGCA AAATGTTACA   1320
   GAATCTAACT GGTGGACATG GCTGTTCATT GTACTGTTTT TTTCTATCTT CTATATGTTT   1380
   AAAAGTATAT AATAAAAATA TTTAATTT                                       1408
(2) INFORMATION FOR SEQ ID NO: 53:
   (i) SEQUENCE CHARACTERISTICS:
      (A) LENGTH: 360 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 53:
   ACCTGACCCA GAGGTCAAGG CTGCAGTGAG ACGAGATTGC CCACTGCCCT CCACCCTGGG     60
   TGATAAGAGT GGGACCCTGT TCAAAACATA CACACACACA CACACACACA CACACACACA    120
   CACACACACA CTCTCTCTCT CTCTCTCTCT CTCTCTCTCT CTCTCTCTCT CTCTCAAAAA    180
   CACTTGGTCT GTTATTTTTC GAAATTGTCA GTCATAGTTA TCTGTTAGAC CAAAGCTGGT    240
   AAGACATTTA TTACATTGCC TCCTACAACT TCATCAGCTA ATGTATTTGC TATATAGCAA    300
   TTACATATGG ATATATTATC TTAGGGGATG GCCAGTATAA AACTGTCACT GAGGAAAGGA    360
(2) INFORMATION FOR SEQ ID NO: 54:
   (i) SEQUENCE CHARACTERISTICS:
      (A) LENGTH: 288 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 54:
   CCTCCCACCT AGCCTCCCCA GTAGCTAGGA CTATAGGCGT GCCCACCAAG CTCAGCTATT     60
   TTTTATTTAG TAGAGACGGG GTTTCGGCAG CTTAGGCCTC GTTCGAACTC CAGTGTGTGT    120
   GTGTGTGTGT GTGTGTGTGT GTGTGTGTGT GTGTGTGTGT GTGTAGATAT TTATTCCCCC    180
   TCCCCCTTGG AAAAGTAAGT AAGCTCCTAC TAGGAATTTA AAACCTGCTT GATCTATATA    240
   AAGACAAACA AGGAAAGACA AACATGGGGG CAGGAAGGAA GGCAGATC                 288
(2) INFORMATION FOR SEQ ID NO: 55:
   (i) SEQUENCE CHARACTERISTICS:
      (A) LENGTH: 141 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 55:
   TCGAGGTAGA TTTGTATTAT ATCCCATGTA CACACACACA CACACACACA CACACACACA    60
   CACACACAGA CTTAATCTGT TTACAGAAAT AAAAGGAATA AAATACCGTT TCTACTATAC   120
   ACCAAAACTA GCCATCTTGA C                                             141
(2) INFORMATION FOR SEQ ID NO: 56:
   (i) SEQUENCE CHARACTERISTICS:
      (A) LENGTH: 305 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 56:
   CCCTGAGAAG GCTTCCTCCT GAGTATGCAT AAACATTCAC AGCTTGCATG CGTGTGTGTG    60
   TGTGTGTGTG TGTGTATGTT TGCTTGCACT GTAAAAACAA TTGCAACATC AACAGAAATA   120
   AAAATTAAAG GAATAATTCT CCTCCGACTC TGCCGTTCCA TCCAGTGAAA CTCTTCATTC   180
   TGGGGTAAAG TTCCTTCAGT TCTTTCATAG ATAGGTATAT ACTTCATAAG TCAAACAATC   240
   AGGCTGGGTG CAGTAGCTCA TGCCTGTAAT CCCAGCCCTT TGGGAGGCCG AGCTGGGCAG   300
   ATCGA                                                               305
(2) INFORMATION FOR SEQ ID NO: 57:
   (i) SEQUENCE CHARACTERISTICS:
      (A) LENGTH: 341 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (iii) HYPOTHETICAL: NO
   (iv) ANTI-SENSE: NO
   (xi) SEQUENCE DESCRIPTION: SEQ ID NO: 57:
   TCCACCCGCC TTGGCCTCCC AAAGCCTGGG ATTACAGGCG TGACTGCCGC ACCCAGCTGT    60
   AAACTGGTTT AATGGTAGAT TTTAGGTATT AACAATAGAT AAAAAGATAC TTTTGGCATA   120
   CTGTGTATTG GGATGGGGTT AGAACAGGTG TCTACCCAAG ACATTTACTT AAAATCGCCC   180
   TCGAAATGCT ATGTGAGCTG TGTGTGTGTG TGTGTGTGTG TGTGTATTAA GGAAAAGCAT   240
   GAAAGTATTT ATGCTTGATT TTTTTTTTTA CTCATAGCTT CATAGTGGAC AGATACATAG   300
   TCTAAATCAA AATGTTTAAA CTTTTTATGT CACTTGCTGT C                       341
(2) INFORMATION FOR SEQ ID NO: 58:
   (i) SEQUENCE CHARACTERISTICS:
      (A) LENGTH: 278 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (xi) SEQUENCE DESCRIPTION: SEQ ID NO: 58:
   Met Ala Met Ser Ser Gly Gly Ser Gly Gly Gly Val Pro Glu Gln Glu
   1               5                   10                  15
   Asp Ser Val Leu Phe Arg Arg Gly Thr Gly Gln Ser Asp Asp Ser Asp
               20                  25                  30
   Ile Trp Asp Asp Thr Ala Leu Ile Lys Ala Tyr Asp Lys Ala Val Ala
           35                  40                  45
   Ser Phe Lys His Ala Leu Lys Asn Gly Asp Ile Cys Glu Thr Ser Gly
       50                  55                  60
   Lys Pro Lys Thr Thr Pro Lys Arg Lys Pro Ala Lys Lys Asn Lys Ser
   65                  70                  75                  80
   Gln Lys Lys Asn Thr Ala Ala Ser Leu Gln Gln Trp Lys Val Gly Asp
                   85                  90                  95
   Lys Cys Ser Ala Ile Trp Ser Glu Asp Gly Cys Ile Tyr Pro Ala Thr
               100                 105                 110
   Ile Ala Ser Ile Asp Phe Lys Arg Glu Thr Cys Val Val Val Tyr Thr
           115                 120                 125
   Gly Tyr Gly Asn Arg Glu Glu Gln Asn Leu Ser Asp Leu Leu Ser Pro
       130                 135                 140
   Ile Cys Glu Val Ala Asn Asn Ile Glu Gln Asn Ala Gln Glu Asn Glu
   145                 150                 155                 160
   Asn Glu Ser Gln Val Ser Thr Asp Glu Ser Glu Asn Ser Arg Ser Pro
                   165                 170                 175
   Gly Asn Lys Ser Asp Asn Ile Lys Pro Lys Ser Ala Pro Trp Asn Ser
               180                 185                 190
   Phe Leu Pro Pro Pro Pro Pro Met Pro Gly Pro Pro Leu Gly Pro Gly
           195                 200                 205
   Lys Pro Gly Leu Lys Phe Asn Gly Pro Pro Pro Pro Pro Pro Pro Pro
       210                 215                 220
   Pro Pro His Leu Leu Ser Cys Trp Leu Pro Pro Phe Pro Ser Gly Pro
   225                 230                 235                 240
   Pro Ile Ile Pro Pro Pro Pro Pro Ile Cys Pro Asp Ser Leu Asp Asp
                   245                 250                 255
   Ala Asp Ala Leu Gly Ser Met Leu Ile Ser Trp Tyr Met Ser Gly Tyr
               260                 265                 270
   His Thr Gly Tyr Tyr Met
           275
(2) INFORMATION FOR SEQ ID NO: 59:
   (i) SEQUENCE CHARACTERISTICS:
      (A) LENGTH: 3246 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 59:
   CCTCCCGGGC ACCGTACTGT TCCGCTCCCA GAAGCCCCGG GCGCCGGAAG TCGTCACTCT     60
   TAAGAAGGGA CGGGGCCCCA CGCTGCGCAC CCGCGGGTTT GCTATGGCGA TGAGCAGCGG    120
   CGGCAGTGGT GGCGGCGTCC CGGAGCAGGA GGATTCCGTG CTGTTCCGGC GCGGCACAGG    180
   CAGGTGAGGT CGCAGCCAGT GCAGTCTCCC TATTAGCGCT CTCAGCACCC TTCTTCCGGC    240
   CCAACTCTCC TTCCGCAGTA ATTTTGTTAT GTGTGGATTA AGATGACTCT TGGTACTAAC    300
   ATACATTTTC TGATTAAACC TATCTGACAT GAGTTGTTTT TATTTCTTAC CCTTTCCAGA    360
   GCGATGATTC TGACATTTGG GATGATACAG CACTGATAAA AGCATATGAT AAAGCTGTGG    420
   CTTCATTTAA GGTATGAAAT GCTTGTTAGT CGTTTTCTTA TTTTCTCGTT ATTCATTTGG    480
   AAAGGAATTG ATAACATACG ATAAAGTGTT AAGTGCTTTC TGAGGTGACG GAGCCTTGAG    540
   ACTAGCTTAT AGTAGTAACT GGGTTATGTC GTGACTTTTA TTCTGTGCAC CACCCTGTAA    600
   CATGTACATT TTTATTCCTA TTTTCGTAGC ATGCTCTAAA GAATGGTGAC ATTTGTGAAA    660
   CTTCGGGTAA ACCAAAAACC ACACCTAAAA GAAAACCTGC TAAGAAGAAT AAAAGCCAAA    720
   AGAAGAATAC TGCAGCTTCC TTACAACAGG TTATTTTAAA ATGTTGAGGA TTTAACTTCA    780
   AAGGATGTCT CATTAGTCCT TATTTAATAG TGTAAAATGT CTTTAACTGC AGGTCGATCA    840
   AAACGAGATG ATAGTTTGCC CTCTTCAAAA GAAATGTGTG CATGTATATA TCTTTGATTT    900
   CTTTTGTAGT GGAAAGTTGG GGACAAATGT TCTGCCATTT GGTCAGAAGA CGGTTGCATT    960
   TACCCAGCTA CCATTGCTTC AATTGATTTT AAGAGAGAAA CCTGTGTTGT GGTTTACACT   1020
   GGATATGGAA ATAGAGAGGA GCAAAATCTG TCCGATCTAC TTTCCCCAAT CTGTGAAGTA   1080
   GCTAATAATA TAGAACAGAA TGCTCAAGAG GTAAGGATAC AAAAAAAAAA AAATTCAATT   1140
   TCTGGAAGCA GAGACTAGAT GAGAAACTGT TAAACAGTAT ACAACCGAGG CATTAATTTT   1200
   TTCTTAATCA CACCCTTATA ACAAAAACCT GCATATTTTT TCTTTTTAAA GAATGAAAAT   1260
   GAAAGCCAAG TTTCAACAGA TGAAAGTGAG AACTCCAGGT CTCCTGGAAA TAAATCAGAT   1320
   AACATCAAGC CCAAATCTGC TCCATGGAAC TCTTTTCTCC CTCCACCACC CCCCATGCCA   1380
   GGGCCAAGAC TGGGACCAGG AAAGGTAAAC CTTCTATGAA AGTTTTCCAG AAAATAGTTA   1440
   ATGTCGGGAC ATTTAACCTC TCTGTTAACT AATTTGTAGC TCTCCAATAT TCTGGGTAAT   1500
   TATTTTTATC CTTTTGGTTT TGAGTCCTTT TTATTCCTAT CATATTGAAA TTGGTAAGTT   1560
   AATTTTCCTT TGAAATATTC CTTATAGCCA GGTCTAAAAT TCAATGGCCC ACCACCGCCA   1620
   CCGCCACCAC CACCACCCCA CTTACTATCA TGCTGGCTGC CTCCATTTCC TTCTGGACCA   1680
   CCAGTAAGTA AAAAAGAGTA TAGGTTAGAT TTTGCTTTCA CATACAATTT GATAATAGAC   1740
   TTTACTTTTT GTTTACTGGA TATAAACAAT ATCTTTTTCT GTCTCCAGAT AATTCCCCCA   1800
   CCACCTCCCA TATGTCCAGA TTCTCTTGAT GATGCTGATG CTTTGGGAAG TATGTTAATT   1860
   TCATGGTACA TGAGTGGCTA TCATACTGGC TATTATATGG TAAGTAATCA CTCAGCATCT   1920
   TTTCCTGACA ATTTTTTTGT AGTTATGTGA CTTTGTTTGG TAAATTTATA AAATACTACT   1980
   CTGCAGCCTA ATAATTGTTT TCTTTGGGAT AACTTTTAAA GTACATTAAA AGACTATCAA   2040
   CTTAATTTCT GATCATATTT TGTTGAATAA AATAAGTAAA ATGTCTTGTG AAACAAAATG   2100
   CTTTTTAACA TCCATATAAA GCTATCTATA TATAGCTATC TATGTCTATA TAGCTATTTT   2160
   TTTTAACTTC CTTTTATTTT CCTTACAGGG TTTCAGACAA AATCAAAAAG AAGGAAGGTG   2220
   CTCACATTCC TTAAATTAAG GAGTAAGTCT GCCAGCATTA TGAAAGTGAA TCTTACTTTT   2280
   GTAAAACTTT ATGGTTTGTG GAAAACAAAT GTTTTTGAAC AGTTAAAAAG TTCAGATGTT   2340
   AAAAAGTTGA AAGGTTAATG TAAAACAATC AATATTAAAG AATTTTGATG CCAAAACTAT   2400
   TAGATAAAAG GTTAATCTAC ATCCCTACTA GAATTCTCAT ACTTAACTGG TTGGTTATGT   2460
   GGAAGAAACA TACTTTCACA ATAAAGAGCT TTAGGATATG ATGCCATTTT ATATCACTAG   2520
   TAGGCAGACC AGCAGACTTT TTTTTATTGT GATATGGGAT AACCTAGGCA TACTGCACTG   2580
   TACACTCTGA CATATGAAGT GCTCTAGTCA AGTTTAACTG GTGTCCACAG AGGACATGGT    2640
   TTAACTGGAA TTCGTCAAGC CTCTGGTTCT AATTTCTCAT TTGCAGGAAA TGCTGGCATA    2700
   GAGCAGCACT AAATGACACC ACTAAAGAAA CGATCAGACA GATCTGGAAT GTGAAGCGTT    2760
   ATAGAAGATA ACTGGCCTCA TTTCTTCAAA ATATCAAGTG TTGGGAAAGA AAAAAGGAAG    2820
   TGGAATGGGT AACTCTTCTT GATTAAAAGT TATGTAATAA CCAAATGCAA TGTGAAATAT    2880
   TTTACTGGAC TCTTTTGAAA AACCATCTAG TAAAAGACTG GGGTGGGGGT GGGAGGCCAG    2940
   CACGGTGGTG AGGCAGTTGA GAAAATTTGA ATGTGGATTA GATTTTGAAT GATATTGGAT    3000
   AATTATTGGT AATTTTATGG CCTGTGAGAA GGGTGTTGTA GTTTATAAAA GACTGTCTTA    3060
   ATTTGCATAC TTAAGCATTT AGGAATGAAG TGTTAGAGTG TCTTAAAATG TTTCAAATGG    3120
   TTTAACAAAA TGTATGTGAG GCGTATGTGG CAAAATGTTA CAGAATCTAA CTGGTGGACA    3180
   TGGCTGTTCA TTGTACTGTT TTTTTCTATC TTCTATATGT TTAAAAGTAT ATAATAAAAA    3240
   TATTTA                                                                3246
(2) INFORMATION FOR SEQ ID NO: 60:
   (i) SEQUENCE CHARACTERISTICS:
      (A) LENGTH: 293 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 60:
   Met Ala Met Ser Ser Gly Gly Ser Gly Gly Gly Val Pro Glu Gln Glu
   1               5                   10                  15
   Asp Ser Val Leu Phe Arg Arg Gly Thr Gly Gln Ser Asp Asp Ser Asp
               20                  25                  30
   Ile Trp Asp Asp Thr Ala Leu Ile Lys Ala Tyr Asp Lys Ala Val Ala
           35                  40                  45
   Ser Phe Lys His Ala Leu Lys Asn Gly Asp Ile Cys Glu Thr Ser Gly
       50                  55                  60
   Lys Pro Lys Thr Thr Pro Lys Arg Lys Pro Ala Lys Lys Asn Lys Ser
   65                  70                  75                  80
   Gln Lys Lys Asn Thr Ala Ala Ser Leu Gln Gln Trp Lys Val Gly Asp
                   85                  90                  95
   Lys Cys Ser Ala Ile Trp Ser Glu Asp Gly Cys Ile Tyr Pro Ala Thr
               100                 105                 110
   Ile Ala Ser Ile Asp Phe Lys Arg Glu Thr Cys Val Val Val Tyr Thr
           115                 120                 125
   Gly Tyr Gly Asn Arg Glu Glu Gln Asn Leu Ser Asp Leu Leu Ser Pro
       130                 135                 140
   Ile Cys Glu Val Ala Asn Asn Ile Glu Gln Asn Ala Gln Glu Asn Glu
   145                 150                 155                 160
   Asn Glu Ser Gln Val Ser Thr Asp Glu Ser Glu Asn Ser Arg Ser Pro
                   165                 170                 175
   Gly Asn Lys Ser Asp Asn Ile Lys Pro Lys Ser Ala Pro Trp Asn Ser
               180                 185                 190
   Phe Leu Pro Pro Pro Pro Met Pro Gly Pro Arg Leu Gly Pro Gly Lys
           195                 200                 205
   Pro Gly Leu Lys Phe Asn Gly Pro Pro Pro Pro Pro Pro Pro Pro Pro
       210                 215                 220
   Pro His Leu Leu Ser Cys Trp Leu Pro Pro Phe Pro Ser Gly Pro Pro
   225                 230                 235                 240
   Ile Ile Pro Pro Pro Pro Pro Ile Cys Pro Asp Ser Leu Asp Asp Ala
                   245                 250                 255
   Asp Ala Leu Gly Ser Met Leu Ile Ser Trp Tyr Met Ser Gly Tyr His
               260                 265                 270
   Thr Gly Tyr Tyr Met Gly Phe Arg Gln Asn Gln Lys Glu Gly Arg Cys
           275                 280                 285
   Ser His Ser Leu Asn
       290
(2) INFORMATION FOR SEQ ID NO: 61:
   (i) SEQUENCE CHARACTERISTICS:
      (A) LENGTH: 637 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 61:
   GATCTGCCTT CCTTCCTGCC CCCATGTTTG TCTTTCCTTG TTTGTCTTTA TATAGATCAA     60
   GCAGGTTTTA AATTCCTAGT AGGAGCTTAC ATTTACTTTT CCAAGGGGGA GGGGGAATAA    120
   ATATCTACAC ACACACACAC ACACACACCA CACTGGAGTT CGAGACGAGG CCTAAGCAAC    180
   ATGCCGAAAC CCCGTCTCTA CTAAATACAA AAAATAGCTG AGCTTGGTGG CGCACGCCTA    240
   TAGTCCTAGC TACTGGGGAG GCTGAGGTGG GAGGATCGCT TGAGCCCAAG AAGTCGAGGC    300
   TGCAGTGAGC CGAGATCGCG CCGCTGCACT CCAGCCTGAG CGACAGGGCG AGGCTCTGTC    360
   TCAAAACAAA CAAACAAAAA AAAAAAGGAA AGGAAATATA ACACAGTGAA ATGAAAGGAT    420
   TGAGAGAAAT GAAAAATATA CACGCCACAA ATGTGGGAGG GCGATAACCA CTCGTAGAAA    480
   GCGTGAGAAG TTACTACAAG CGGTCCTCCC GGGCACCGTA CTGTTCCGCT CCCAGAAGCC    540
   CCGGGCGCCG GAAGTCGTCA CTCTTAAGAA GGGACGGGGC CCCACGCTGC GCACCCGCGG    600
   GTTTGCTATG GCGATGAGCA GCGGCGGCAG TGGTGGC                             637
(2) INFORMATION FOR SEQ ID NO: 62:
   (i) SEQUENCE CHARACTERISTICS:
      (A) LENGTH: 10 amino acids
      (B) TYPE: amino acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (xi) SEQUENCE DESCRIPTION: SEQ ID NO: 62:
   Met Ala Met Ser Ser Gly Gly Ser Gly Gly
   1               5                   10
(2) INFORMATION FOR SEQ ID NO: 63:
   (i) SEQUENCE CHARACTERISTICS:
      (A) LENGTH: 288 amino acids
      (B) TYPE: amino acid
      (C) STRANDEDNESS: double
      (D) TOPOLOGY: linear
   (ii) MOLECULE TYPE: protein
   (iii) HYPOTHETICAL: NO
   (iv) ANTI-SENSE: NO
   (xi) SEQUENCE DESCRIPTION: SEQ ID NO: 63:
   Met Ala Met Gly Ser Gly Gly Ala Gly Ser Glu Gln Glu Asp Thr Val
   1               5                   10                  15
   Leu Phe Arg Arg Gly Thr Gly Gln Ser Asp Asp Ser Asp Ile Trp Asp
               20                  25                  30
   Asp Thr Ala Leu Ile Lys Ala Tyr Asp Lys Ala Val Ala Ser Phe Lys
           35                  40                  45
   His Ala Leu Lys Asn Gly Asp Ile Cys Glu Thr Pro Asp Lys Pro Lys
       50                  55                  60
   Gly Thr Ala Arg Arg Lys Pro Ala Lys Lys Asn Lys Ser Gln Lys Lys
   65                  70                  75                  80
   Asn Ala Thr Thr Pro Leu Lys Gln Trp Lys Val Gly Asp Lys Cys Ser
                   85                  90                  95
   Ala Val Trp Ser Glu Asp Gly Cys Ile Tyr Pro Ala Thr Ile Thr Ser
               100                 105                 110
   Ile Asp Phe Lys Arg Glu Thr Cys Val Val Val Tyr Thr Gly Tyr Gly
           115                 120                 125
   Asn Arg Glu Glu Gln Asn Leu Ser Asp Leu Leu Ser Pro Thr Cys Glu
       130                 135                 140
   Val Ala Asn Ser Thr Glu Gln Asn Thr Gln Glu Asn Glu Ser Gln Val
   145                 150                 155                 160
   Ser Thr Asp Asp Ser Glu His Ser Ser Arg Ser Leu Arg Ser Lys Ala
                   165                 170                 175
   His Ser Lys Ser Lys Ala Ala Pro Trp Thr Ser Phe Leu Pro Pro Pro
               180                 185                 190
   Pro Pro Met Pro Gly Ser Gly Leu Gly Pro Gly Lys Pro Gly Leu Lys
           195                 200                 205
   Phe Asn Gly Pro Pro Pro Pro Pro Pro Leu Pro Pro Pro Pro Phe Leu
       210                 215                 220
   Pro Cys Trp Met Pro Pro Phe Pro Ser Gly Pro Pro Ile Ile Pro Pro
   225                 230                 235                 240
   Pro Pro Pro Ile Ser Pro Asp Cys Leu Asp Asp Thr Asp Ala Leu Gly
                   245                 250                 255
   Ser Met Leu Ile Ser Trp Tyr Met Ser Gly Tyr His Thr Gly Tyr Tyr
               260                 265                 270
   Met Gly Phe Arg Gln Asn Lys Lys Glu Gly Lys Cys Ser His Thr Asn
           275                 280                 285
(2) INFORMATION FOR SEQ ID NO: 64:
   (i) SEQUENCE CHARACTERISTICS:
      (A) LENGTH: 885 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 64:
   CGGCGTGGTA GCAGGCCATG GCGATGGGCA GTGGCGGAGC GGGCTCCGAG CAGGAAGATA 60
   CGGTGCTGTT CCGGCGTGGC ACCGGCCAGA GTGATGATTC TGACATTTGG GATGATACAG 120
   CATTGATAAA AGCTTATGAT AAAGCTGTGG CTTCCTTTAA GCATGCTCTA AAGAACGGTG 180
   ACATTTGTGA AACTCCAGAT AAGCCAAAAG GCACAGCCAG AAGAAAACCT GCCAAGAAGA 240
   ATAAAAGCCA AAAGAAGAAT GCCACAACTC CCTTGAAACA GTGGAAAGTT GGTGACAAGT 300
   GTTCTGCTGT TTGGTCAGAA GACGGCTGCA TTTACCCAGC TACTATTACG TCCATTGACT 360
   TTAAGAGAGA AACCTGTGTC GTGGTTTATA CTGGATATGG AAACAGAGAG GAGCAAAACT 420
   TATCTGACCT ACTTTCCCCG ACCTGTGAAG TAGCTAATAG TACAGAACAG AACACTCAGG 480
   AGAATGAAAG TCAAGTTTCC ACAGACGACA GTGAACACTC CTCCAGATCG CTCAGAAGTA 540
   AAGCACACAG CAAGTCCAAA GCTGCTCCGT GGACCTCATT TCTTCCTCCA CCACCCCCAA 600
   TGCCAGGGTC AGGATTAGGA CCAGGAAAGC CAGGTCTAAA ATTCAACGGC CCGCCGCCGC 660
   CGCCTCCACT ACCCCCTCCC CCCTTCCTGC CGTGCTGGAT GCCCCCGTTC CCTTCAGGAC 720
   CACCAATAAT CCCGCCACCC CCTCCCATCT CTCCCGACTG TCTGGATGAC ACTGATGCCC 780
   TGGGCAGTAT GCTAATCTCT TGGTACATGA GTGGCTACCA CACTGGCTAC TATATGGGTT 840
   TCAGACAAAA TAAAAAAGAA GGAAAGTGCT CACATACAAA TTAAG 885
(2) INFORMATION FOR SEQ ID NO: 65:
   (i) SEQUENCE CHARACTERISTICS:
      (A) LENGTH: 289 amino acids
      (B) TYPE: amino acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 65:
   Gly Ser Gly Gly Gly Val Pro Glu Gln Glu Asp Ser Val Leu Phe Arg
   1               5                   10                  15
   Arg Gly Thr Gly Gln Ser Asp Asp Ser Asp Ile Trp Asp Asp Thr Ala
               20                  25                  30
   Leu Ile Lys Ala Tyr Asp Lys Ala Val Ala Ser Phe Lys His Ala Leu
           35                  40                  45
   Lys Asn Gly Asp Ile Cys Glu Thr Ser Gly Lys Pro Lys Thr Thr Pro
       50                  55                  60
   Lys Arg Lys Pro Ala Lys Ala Lys Lys Asn Lys Ser Gln Lys Lys Asn
   65                  70                  75                  80
   Thr Ala Ala Ser Leu Gln Gln Trp Lys Val Gly Asp Lys Cys Ser Ala
                   85                  90                  95
   Ile Trp Ser Glu Asp Gly Cys Ile Tyr Pro Ala Thr Ile Ala Ser Ile
               100                 105                 110
   Asp Phe Lys Arg Glu Thr Cys Val Val Val Tyr Thr Gly Tyr Gly Asn
           115                 120                 125
   Arg Glu Glu Gln Asn Leu Ser Asp Leu Leu Ser Pro Ile Cys Glu Val
       130                 135                 140
   Ala Asn Asn Ile Glu Gln Asn Ala Gln Glu Asn Glu Asn Glu Ser Gln
   145                 150                 155                 160
   Val Ser Thr Asp Glu Ser Glu Asn Ser Arg Ser Pro Gly Asn Lys Ser
                   165                 170                 175
   Asp Asn Ile Lys Pro Lys Ser Ala Pro Trp Asn Ser Phe Leu Pro Pro
               180                 185                 190
   Pro Pro Pro Met Pro Gly Pro Arg Leu Gly Pro Gly Lys Pro Gly Leu
           195                 200                 205
   Lys Phe Asn Gly Pro Pro Pro Pro Pro Pro Pro Pro Pro Pro His Leu
       210                 215                 220
   Leu Ser Cys Trp Leu Pro Pro Phe Pro Ser Gly Pro Pro Ile Ile Pro
   225                 230                 235                 240
   Pro Pro Pro Pro Ile Cys Pro Asp Ser Leu Asp Asp Ala Asp Ala Leu
                   245                 250                 255
   Gly Ser Met Leu Ile Ser Trp Tyr Met Ser Gly Tyr His Thr Gly Tyr
               260                 265                 270
   Tyr Met Gly Phe Arg Gln Asn Gln Lys Glu Gly Arg Cys Ser His Ser
           275                 280                 285
   Leu
(2) INFORMATION FOR SEQ ID NO: 66:
   (i) SEQUENCE CHARACTERISTICS:
      (A) LENGTH: 285 amino acids
      (B) TYPE: amino acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (xi) SEQUENCE DESCRIPTION: SEQ ID NO: 66:
   Gly Ser Gly Gly Ala Gly Ser Glu Gln Glu Asp Thr Val Leu Phe Arg
   1               5                   10                  15
   Arg Gly Thr Gly Gln Ser Asp Asp Ser Asp Ile Trp Asp Asp Thr Ala
               20                  25                  30
   Leu Ile Lys Ala Tyr Asp Lys Ala Val Ala Ser Phe Lys His Ala Leu
           35                  40                  45
   Lys Asn Gly Asp Ile Cys Glu Thr Pro Asp Lys Pro Lys Gly Thr Ala
       50                  55                  60
   Arg Arg Lys Pro Ala Lys Lys Asn Lys Ser Gln Lys Lys Asn Ala Thr
   65                  70                  75                  80
   Thr Pro Leu Lys Gln Trp Lys Val Gly Asp Lys Cys Ser Ala Val Trp
                   85                  90                  95
   Ser Glu Asp Gly Cys Ile Tyr Pro Ala Thr Ile Thr Ser Ile Asp Phe
               100                 105                 110
   Lys Arg Glu Thr Cys Val Val Val Tyr Thr Gly Tyr Gly Asn Arg Glu
           115                 120                 125
   Glu Gln Asn Leu Ser Asp Leu Leu Ser Pro Thr Cys Glu Val Ala Asn
       130                 135                 140
   Ser Thr Glu Gln Asn Thr Gln Glu Asn Glu Ser Gln Val Ser Thr Asp
   145                 150                 155                 160
   Asp Ser Glu His Ser Ser Arg Ser Leu Arg Ser Lys Ala His Ser Lys
                   165                 170                 175
   Ser Lys Ala Ala Pro Trp Thr Ser Phe Leu Pro Pro Pro Pro Pro Met
               180                 185                 190
   Pro Gly Ser Gly Leu Gly Pro Gly Lys Pro Gly Leu Lys Phe Asn Gly
           195                 200                 205
   Pro Pro Pro Pro Pro Pro Leu Pro Pro Pro Pro Phe Leu Pro Cys Trp
       210                 215                 220
   Met Pro Pro Phe Pro Ser Gly Pro Pro Ile Ile Pro Pro Pro Pro Pro
   225                 230                 235                 240
   Ile Ser Pro Asp Cys Leu Asp Asp Thr Asp Ala Leu Gly Ser Met Leu
                   245                 250                 255
   Ile Ser Trp Tyr Met Ser Gly Tyr His Thr Gly Tyr Tyr Met Gly Phe
               260                 265                 270
   Arg Gln Asn Lys Lys Glu Gly Lys Cys Ser His Thr Asn
           275                 280                 285

## Description

### BACKGROUND OF THE INVENTION

### 1. Field of the Invention

The present invention relates to the discovery of the human survival motor-neuron gene or SMN gene which is a chromosome 5-SMA (Spinal Muscular Atrophy) determining gene. The present invention further relates to the nucleotide sequence encoding the SMN gene and corresponding amino acid sequence, a vector containing the gene encoding the SMN protein or a DNA sequence corresponding to the gene and transformant strains containing the SMN gene or a DNA sequence corresponding to the gene.

More particularly, the present invention relates to means and methods for detecting motor neuron diseases having symptoms of muscular weakness with or without sensory changes such as amytrophic lateral sclerosis (ALS), spinal muscular atrophy (SMA), primary lateral sclerosis (PLS), arthrogryposis multiplex congenita (AMC), and the like. The methods for detecting such motor neuron diseases include, but are not limited to, the use of specific DNA primers in the PCR technique, the use of hybridization probes and the use of polyclonal and monoclonal antibodies.

Even more particularly, the present invention relates to the use of the human SMN gene or part of the gene, cDNA, oligonucleotide or the encoded protein or part thereof in therapy by insertion of the human SMN gene or part of the gene, cDNA, oligonucleotide or the encoded protein or part thereof, if required, into engineered viruses or vectors that serve as harmless carriers to transport the gene or part of the gene, cDNA, oligonucleotide or the encoded protein or part thereof to the body's cells including bone marrow cells.

The invention further relates to antigen sequences directed to the SMN gene.

In order to provide means for the therapy of motor neuron diseases, the invention also relates to the protein encoded by the SMN gene.

The present invention also relates to the isolation of the mouse SMN gene, the nucleotide sequence encoding the mouse SMN gene and corresponding amino acid sequence. A transgenic mouse model that hyperexpresses all or part of the SMN gene and a transgenic mouse model produced by homologous recombination with a mutated SMN gene is also described.

### 2. State of the Art

Degenerative motor neuron diseases can be placed into three major categories. Amyotrophic lateral sclerosis or ALS, motor neuron diseases such as spinal muscular atrophy (SMA) and motor neuron diseases associated with other degenerative disorders such as primary lateral sclerosis (PLS).

Amyotrophic lateral sclerosis (ALS) is the most frequently encountered form of progressive neuron disease and is characteristically a disorder of middle age. The disease is characterized by progressive loss of motor neurons, both in the cerebral cortex and in the anterior horns of the spinal cord, together with their homologues in some motor nuclei of the brainstem. It typically affects both upper and lower motor neurons, although variants may predominantly involve only particularly subsets of motor neurons, particularly early in the course of illness.

ALS is evidenced by the development of asymmetric weakness, with fatigue and cramping of affected muscles. The weakness is accompanied by visible wasting and atrophy of the muscles evolves and over time, more and more muscles become involved until the disorder takes on a symmetric distribution in all regions, including muscles of chewing, swallowing and movement of the face and tongue. Fifty percent of patients having ALS can be expected to die within three to five years from the onset of the disease. Presently, there is no treatment that has influence on the pathologic process of ALS.

Spinal muscular atrophies (SMA) are characterized by degeneration of anterior horn cells of the spinal cord leading to progressive symmetrical limb and trunk paralysis associated with muscular atrophy. SMA represents the second most common fatal, autosomal recess e disorder after cystic fibrosis (1 out 6000 newborns). Childhood SMA is classically subdivided into three clinical groups on the basis of age of onset and clinical course. The acute form of Werdnig-Hoffmann disease (Type I) is characterized by severe generalized muscle weakness and hypotonia at birth or in the 3 months following birth. Death, from respiratory failure, usually occurs within the first two years. This disease may be distinguished from the intermediate (Type II) and juvenile (Type III, Kugelberg-Welander disease) forms. Type II children were able to sit but unable to stand or walk unaided, and they live beyond 4 years. Type III patients had proximal muscle weakness, starting after the age of two. The underlying biochemical defect remains unknown. In addition there is known to exist a slowly evolving adult form of SMA, sometimes referred to as SMA IV.

Primary lateral slcerosis (PLS) is a variant of ALS and occurs as a sporadic disease of late life. Neuropathologically in PLS there is a degeneration of the corticospinal (pyramidal) tracts, which appear almost normal at brainstem levels but become increasingly atrophic as they descend through the spinal column. The lower limbs are affected earliest and most severely.

Arthrogryposis Multiplex Congenita (AMC) is a frequent syndrome characterized by congenital joint fixation (incidence of 1 out of 3000 live births) resulting from decreased fetal movements *in utero* (Stem, W.G., JAMA, 81:1507-1510 (1923) ; Hall, J.G., Clin. Orthop., 194:44-53 (1985)). AMC has been ascribed to either oligo-hydramnios or a variety of diseases involving the central nervous system, skeletal muscle, or spinal cord. Since neuronal degeneration and neuronophagia occur in the anterior horns, it has been hypothesized that the AMC of neurogenic origin could be related to acute spinal muscular atrophy; SMA Type I Werdnig-Hoffman disease (Banker, B.Q., Hum. Pathol., (1986); 117:656-672).

The detection and clinical diagnosis for ALS, AMC, SMA and PLS is quite limited to muscle biopsies, the clinical diagnosis by a physician and electromyography (EMG). For example, the clinical criteria for diagnosing SMA is set forth in the Clinical Criteria International SMA Consortium (Munsat T.L., Neuromuscular Disorders, Vol. 1, p. 81 (1991)). But due to the complications of the various tests to detect motor neuron disorders, the clinician usually attempts to eliminate various categories of other disease states such as structural lesions, infections, intoxications, metabolic disorders and heriditary biochemical disorders prior to utilizing the above-described test methods.

Presently there is no treatment for any of the above-mentioned motor neuron disorders. Basic rehabilitative measures, including mechanical aids of various kinds, may help patients that have these diseases overcome the effects of their disabilities, but often confining respiratory support systems are necessary to have the patient survive longer.

Accordingly, it is an object of the present invention to characterize the SMA gene which is responsible for SMA disorders and to clone the SMA gene into a vector, for example a plasmid, a cosmid, a phage, a YAC vector, that can be used in the transformation process to produce large quantities of the SMN gene and SMN protein.

In yet another aspect of the invention is the use of primers and hybridization probes to detect and diagnose patients having motor neuron disorders such as AMC, ALS, SMA and PLS. Yet another aspect of the present invention is the use of the SMN gene or part thereof or cDNA, oligonucleotides, protein or part thereof in therapy to correct disorders present in, for example AMC, SMA, ALS and PLS patients, especially gene disorders.

In yet another aspect, the present invention provides monoclonal and polyclonal antibodies for detection of SMN gene defects in SMA patients.

According to a further aspect of the invention, the therapy of motor neuron diseases can involve the protein encoded by the SMN gene.

These and other objects are achieved by the present invention as evidenced by the summary of the invention, the description of the preferred embodiments and the claims.

### OBJECTS AND SUMMARY OF THE INVENTION

It is an object of the present invention to provide a novel human Survival Motor Neuron gene or SMN gene, its DNA sequence and amino acid sequence.

Yet another aspect of the present invention is the provision of a vector which is capable of replicating in a host microorganism to provide large quantities of the human SMN protein.

Yet another aspect of the present invention is the provision of specific DNA sequences that can be used to detect and diagnose spinal muscular atrophy and other motor neuron disorders. These DNA sequences can be used as primers in the polymerase chain reaction to amplify and detect the SMN gene sequence, a truncated or mutated version of the SMN gene sequence or lack of said sequence which leads to the diagnosis of SMA, AMC, and other motor neuron disorders.

Yet another aspect of the present invention provides a transgenic mouse that hyperexpresses all or part of the SMN gene or a transgenic mouse that by homologous recombination with a mutated mouse SMN gene produces abnormalities in the SMN gene is also described.

The inventors have identified two genes respectively designated T-BCD541 and C-BCD541, which are involved in motor neuron disorders.

The T-BCD541 gene is responsible for the motor neuron diseases of the SMA type, since its alteration either by partial or total deletion, by mutation or any other modification, is sufficient to lead to a pathological state at the clinical electromyographic or muscle morphological levels.

The C-BCD541 gene is different from the T-BCD541 gene, at the level of the cDNA, since two nucleotides are modified. This C-BCD541 gene is nevertheless not correctly processed during the transcription in controls and patients suffering from motor neuron diseases. The genomic DNA of the C-BCD541 gene is not correctly spliced during the transcription providing thus for an abnormal transcript. The difference between the splicing of the T-BCD541 and the C-BCD541 gene results from differences in the sequence of the introns of these genes.

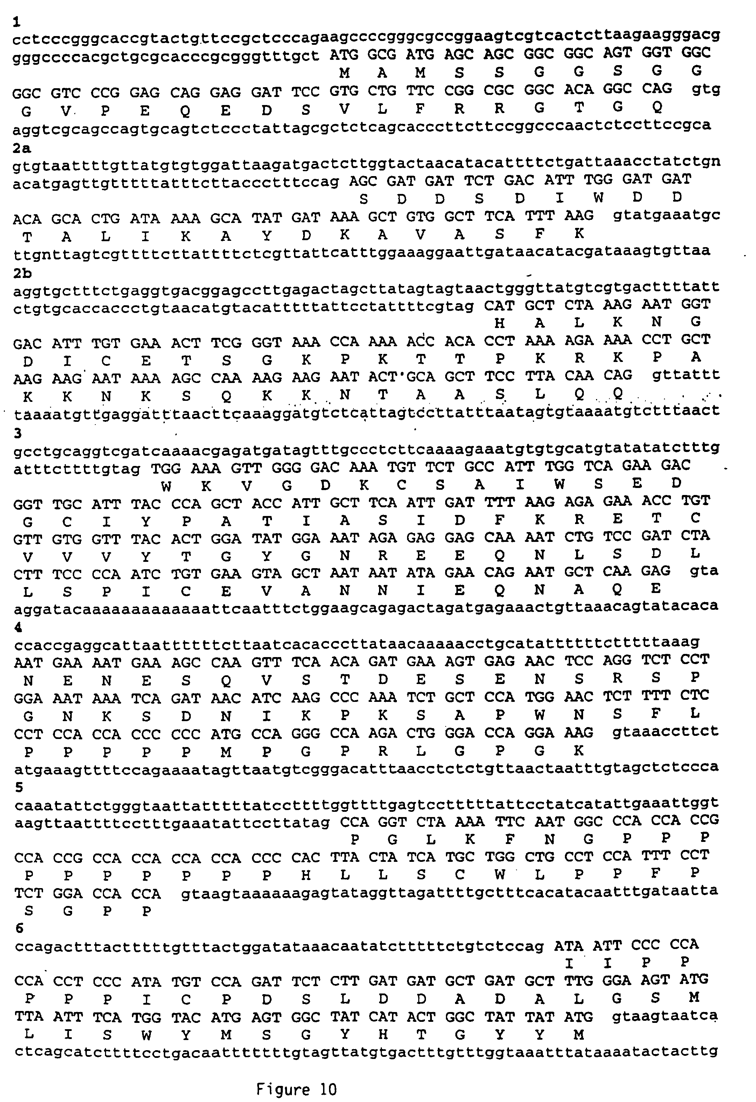

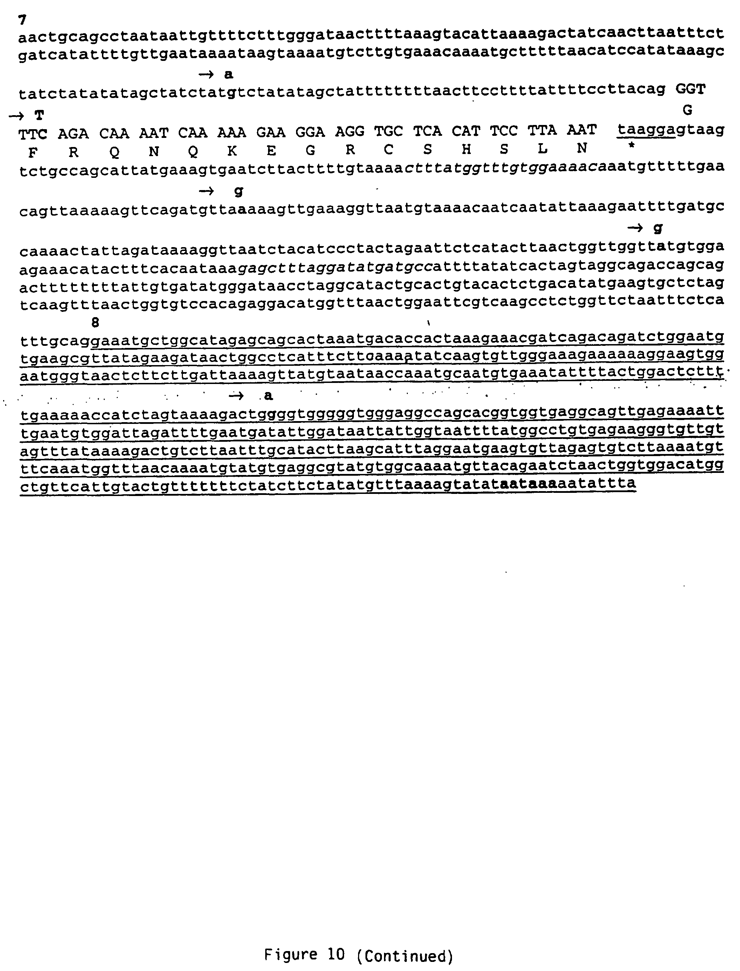

The present invention thus further characterizes the structure and organization of the human SMN gene which was found to be approximately 20 kb in length and consists of 9 exons interrupted by 8 introns. The nucleotide sequence, amino acid sequence as well as the exon-intron boundaries of the human SMN gene is set forth in Figure 10. All exon-intron boundaries display the consensus sequence found in other human genes. A polyadenylation consensus site is localized about 550 bp downstream from the stop codon (Figure 10). The entire intron/exon structure of the SMN gene permits the characterizations of the SMN gene mutations in SMA disease or other motor neuron diseases.

The present invention also defines means for the detection of genomic abnormalities relating to motor neuron diseases at the level of the T-BCD541 gene or at the level of the C-BCD541 gene.

The genes of the invention can be further defined in that each of them comprise intronic sequences corresponding to the following sequences :
In the T-BCD541 gene
- for intron n° 6 :
- for intron n° 7 :
In the C-BCD541 gene :
- for intron n° 6 :
- for intron n° 7 :

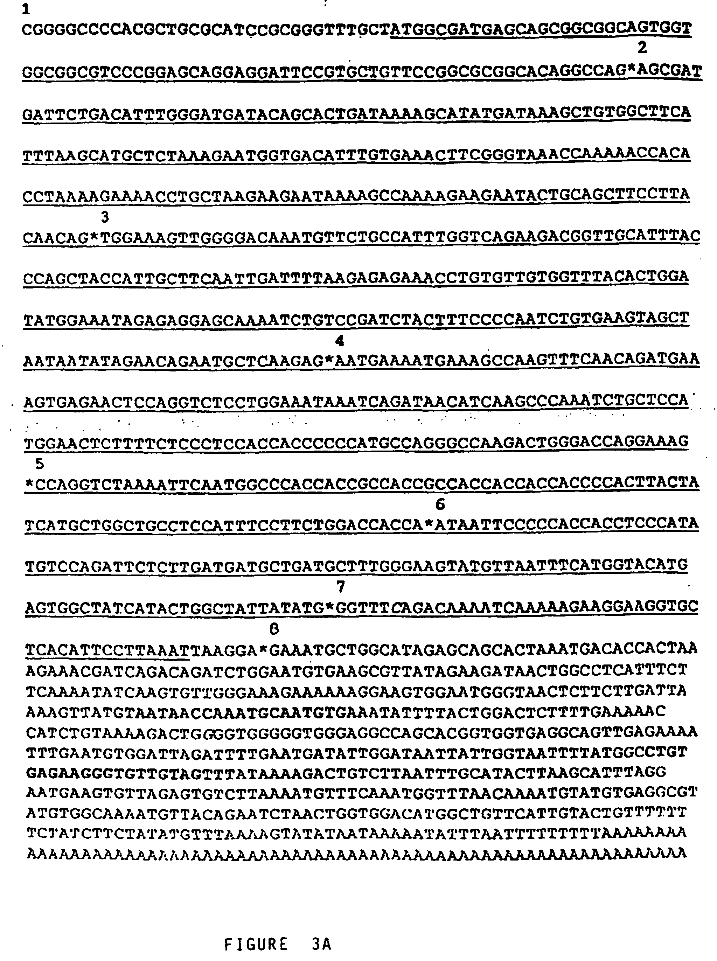

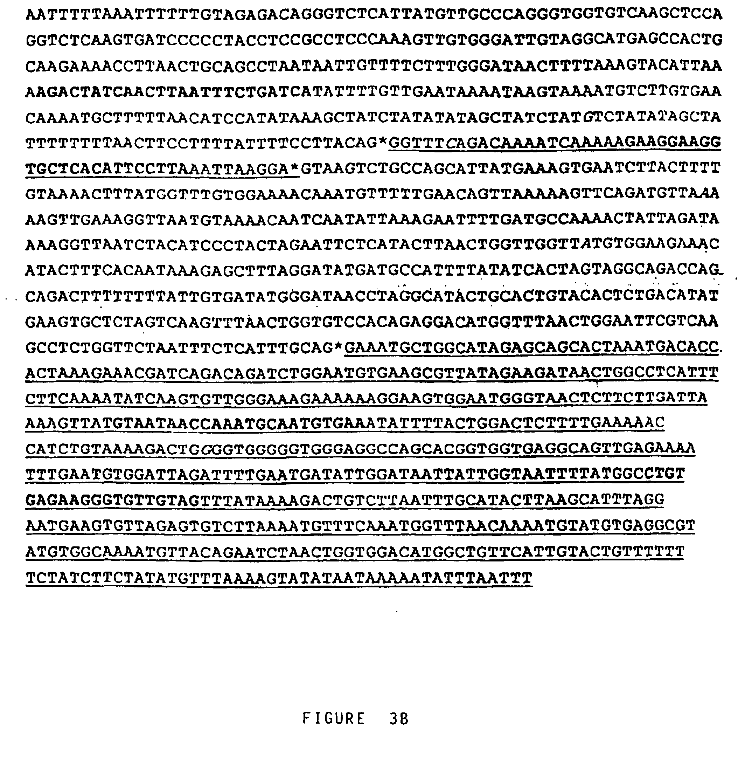

In a preferred embodiment of the invention, the gene of the invention is capable of hybridizing in stringent conditions with the sequence of Figure 3 used as probe.

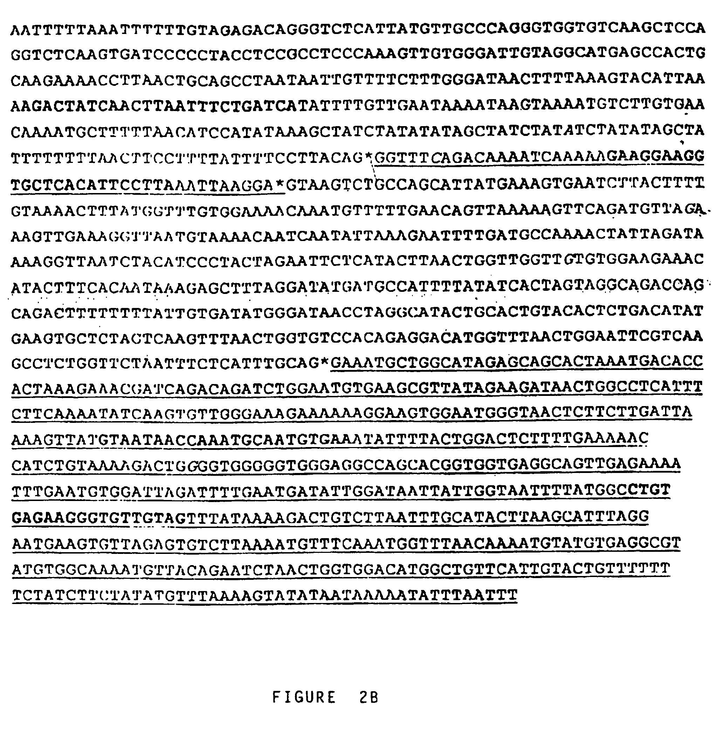

As hereabove written, the invention further relates to a variant of the SMN gene, which variant is a C-BCD541 gene having a cDNA sequence corresponding to the sequence of Figure 2.

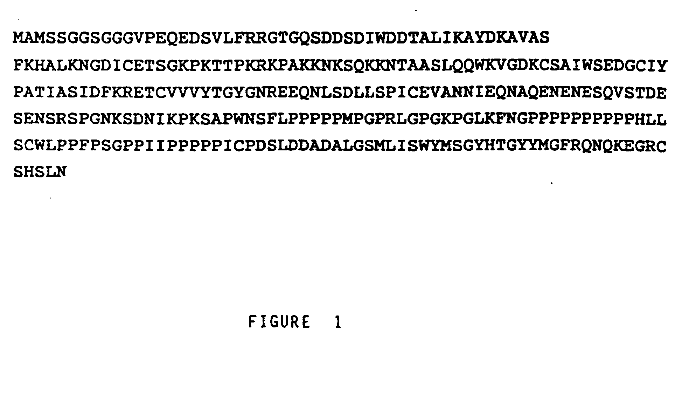

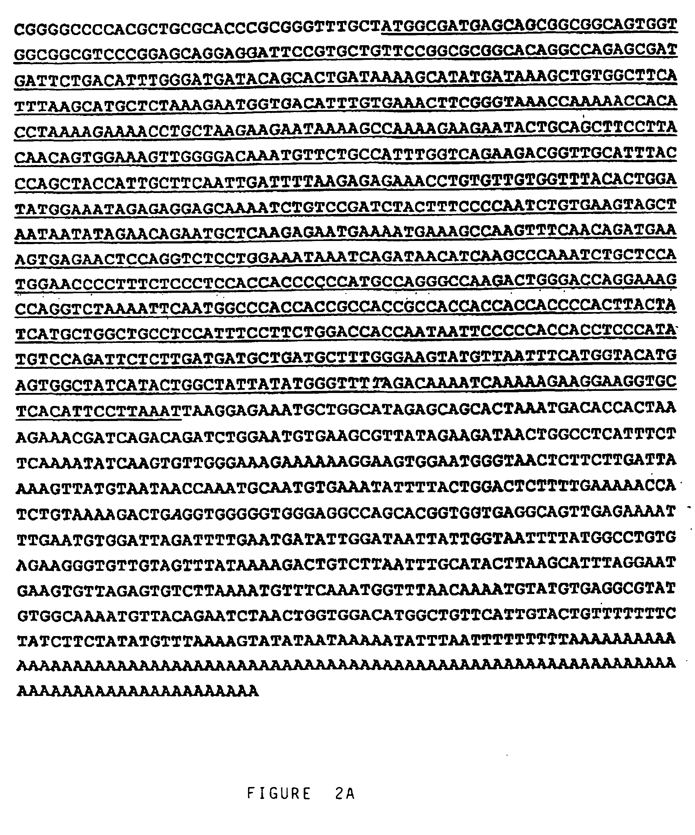

The invention also relates to cDNA sequences such as obtained from one of the above genes. Such cDNA sequences are disclosed in Figures 2 and 3. Both of these cDNA sequence are capable of encoding a protein comprising the amino acid sequence described on Figure 1.

Despite this capacity to encode for such a protein, the inventors have noted that the C-BCD541 gene is able to produce in vivo this protein or is not able to produce it in a sufficient quantity due to the abnormal splicing of the gene during the transcription. Thus, the presence of the C-BCD541 gene does not enable to correct in vivo the deficiency (deletion, mutation,...) of the T-BCD541 gene responsible for the motor neuron diseases of the SMA type or other motor neuron disorders.

In a particular embodiment, the invention relates also to a nucleotide sequence comprising nucleotides 34 to 915 of the sequence of Figure 3, or to a sequence comprising nucleotides 34 to 915 of the sequence of Figure 2.

These nucleotide sequences correspond to the coding sequence of respectively the T-BCD541 gene and C-BCD541 gene.

The introns of the hereabove described genes are also included in the application. Especially introns 6 and 7 have respectively the following sequences:
For the T-BCD541 gene :
- Intron 6 :
- Intron 7 :
For the C-BCD541 gene:
- Intron 6 :
- Intron 7 :

The invention further encompasses a nucleotide sequence, characterized in that it comprises at least around 9 nucleotides and in that it is comprised within a sequence which has been described above

For the determination of the hybridization conditions, reference is made to the hybridization techniques for oligonucleotides probes such as disclosed in Sambrook et al, Molecular Cloning, a Laboratory Manual, 2nd edition, 1989.

The sequences of the invention are either DNA (especially genomic DNA or cDNA or synthetic DNA) or RNA They can be used as probes for the detection of the T-BCD541 or C-BCD541 genes or as primers for the amplification of genomic DNA present in a biological sample.

Preferred primers are those comprising or relating to the following sequences:
a)
b)

The above primers are characteristic of exon 7 of the T-BCD541 gene.
(c)
(d)
   The above primers are characteristic of exon 8 of the T-BCD541 gene.

The primers used by pairs can form sets for the amplification of genomic DNA in order to detect motor neuron diseases.

Inverted complementary sequences with respect to the above primers can also be used.

Preferred sets of primers are the following :
- a pair of primers contained in the sequence comprising nucleotides 921 to 1469 of the sequence of Figure 3 and/or
- a pair of primers comprising the following sequences :

Another preferred set of primers comprises :
- a pair of primers having the following sequences :
- a pair of primers having the following sequences : and/or

From a general point of view for the detection of divergence in exon 7, between the T-BCD541 and C-BCD541 genes oligonucleotide primers can be selected in the fragment 5' from the divergence and within exon 7 or intron 7.

Other primers that can be used for SSCP analysis for diagnostic purposes are selected from amongst the following :

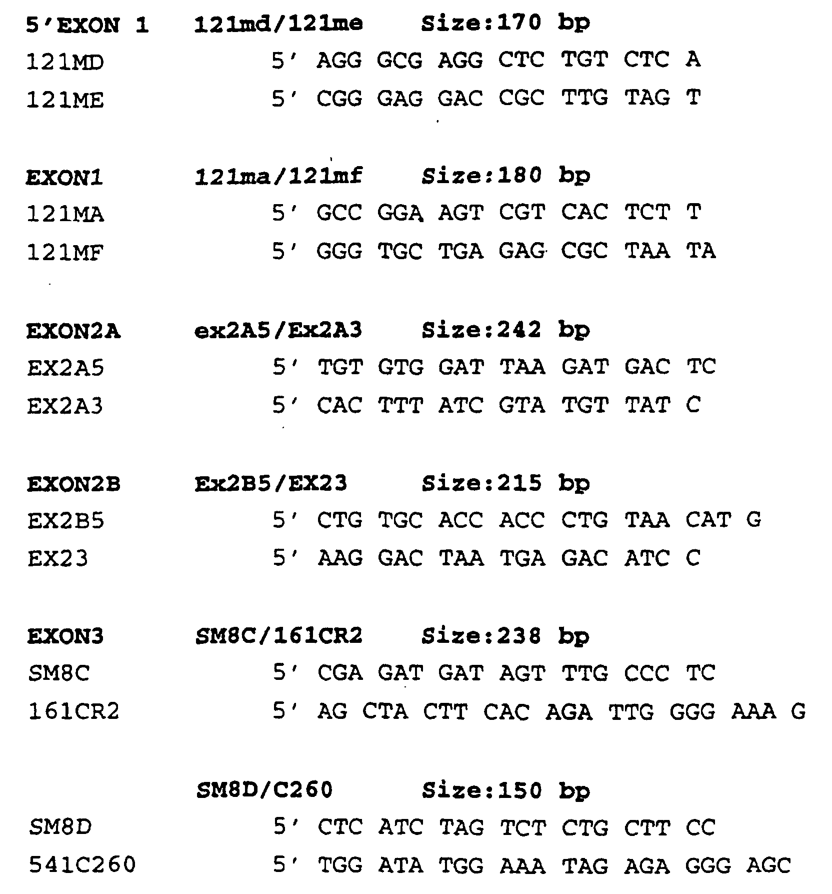

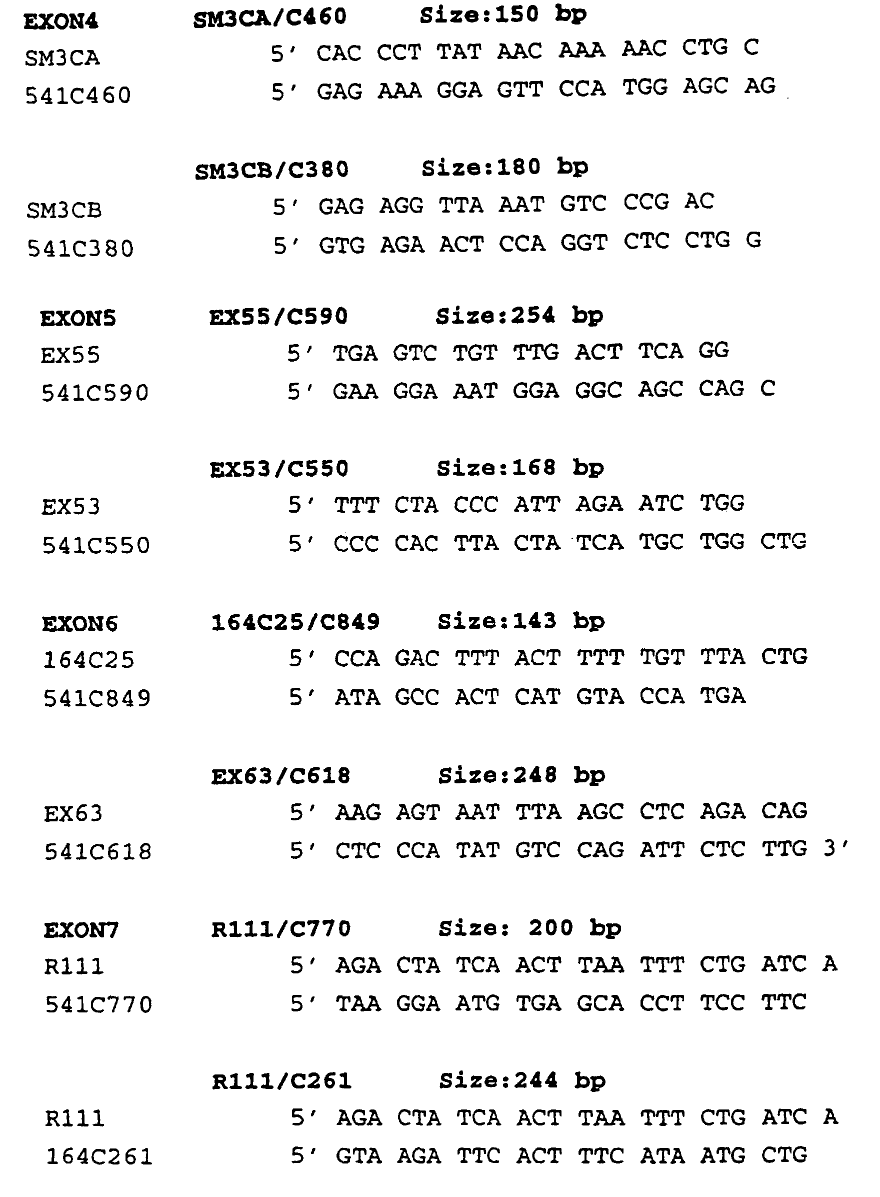

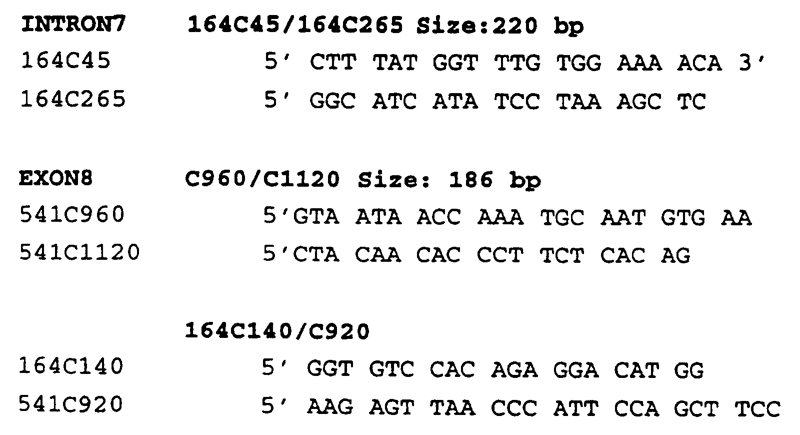

The invention also concerns antisense DNA or RNA, capable of hybridizing with the C-BCD541 gene and particularly to the intron sequences, especially with the fragment of the introns which differ from the corresponding part in the T-BCD541 gene.

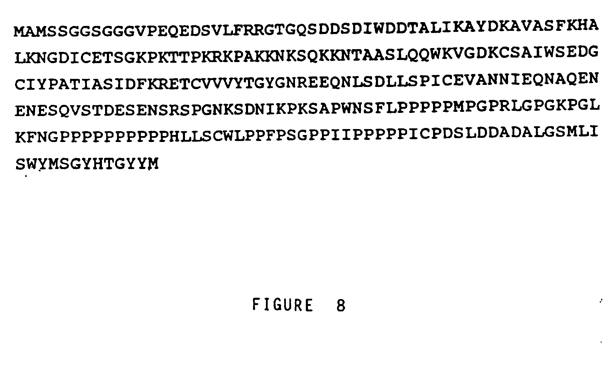

The invention also relates to a protein comprising the amino acid sequence of Figure 1, or to a protein having the amino acid sequence of Figure 8.

The protein relating to the sequence of Figure 1 can be used in a composition for the treatment of motor neuron diseases, via oral, intra-muscular, intravenous administration, or via administration in the spinal cord fluid.

The invention further provides a kit for the in vitro diagnosis of motor neuron diseases, comprising :
- a set of primers as described above ;
- reagents for an amplification reaction ; and
- a probe for the detection of the amplified product.

According to another embodiment of the invention, a kit for the detection of the motor neuron diseases containing a hybridization probe as described above is provided.

Oligonucleotide probes corresponding to the divergences between the genes can be used.

The diagnosis can be especially directed to SMA motor neuron pathology.

The invention also concerns cloning or expression vectors comprising a nucleotide sequence as defined above. Such vectors can be, for example, plasmids, cosmids, phages, YAC, pYAC, and the like. Preferably, such a vector has a motor neuron tropism. Especially for the purpose of defining means for gene therapy, it can be chosen among poliovirus vector, herpes virus, adenovirus, retrovirus vectors, synthetic vectors and the like.

The invention also concerns recombinant host cells, i.e., yeasts, CHO cells, baculovirus, bone marrow cells, *E. coli*, fibroblasts-epithelial cells, transformed by the above recombinant sequences.

The invention also relates to a method for detecting motor neuron disorders including spinal muscular atrophy, amyotrophic lateral sclerosis and primary lateral sclerosis, said method comprising the steps of :
(a) extracting DNA from a patient sample ;
(b) amplifying said DNA with primers as described above ;
(c) subjecting said amplified DNA to SCCP ;
(d) autoradiographing the gels ; and
(e) detecting the presence or absence of the motor neuron disorder.

Steps (c) and (d) can be replaced by a step of digestion with BsrI enzyme or with any other enzyme capable of recognizing specifically the divergence of the genes or mismatches in genes, or by sequencing.

The invention also relates to a method for detecting spinal muscular atrophy, said method comprising the steps of :
(a) extracting DNA from a patient sample ;
(b) hybridizing said DNA with a DNA probe comprising all or part of the cDNA sequence of Figure 3 or of Figure 2 under stringent conditions; and
(c) detecting the hybrids possibly formed.

The invention also relates to a method for detecting arthrogryposis multiplex congenita, said method comprising the steps of :
(a) extracting DNA from a patient sample;
(b) amplifying said DNA via PCR using unlabeled primers from exon 7 and exon 8 of the SMN gene ;
(c) subjecting said amplified DNA to SCCP ;
(d) autoradiographing the gels ; and
(e) detecting the presence of arthrogryposis multiplex congenita.

Yet another method to detect arthrogryposis multiplex congenita concerns dinucleotide Repeat Polymorphism Analysis using genotyping markers C272 and C212 after PCR amplification.

The present invention further concerns polyclonal antiserum or monocional antibodies directed to the protein of Figure 1, the protein of Figure 8 or the protein of Figure 12.

Yet another aspect of the present invention is directed to the use of the entire or partial nucleotide sequence of SMN as a probe to detect SMA as well as to indetify and clone genes related to SMN gene motor neuron in animals or organisms.

Yet another aspect of the present invention is the use of the SMA protein to produce polyclonal and monoclonal antibodies, which antibodies may be used to detect and diagnose SMA.

In another aspect, polyclonal rabbit antiserum were generated against synthetic peptides corresponding to the amino acid sequence of Figures 1, 8 and 12, including the amino acid terminus and the carboxy terminus.

Accordingly, in one of its process aspects, the present invention relates to the detection of SMA in patients having SMA or related motor neuron disorders such as AMC, ALS and PLS.

Yet another aspect of the present invention is to administer the SMN gene part thereof, cDNA or oligonucleotides to patients who are either lacking the gene or have a genetically defective gene as such or after incorporation into engineered viruses or vectors.

These and other aspects of the present invention will be discussed in detail below in the preferred embodiments of the present invention.

### BRIEF DESCRIPTION OF THE DRAWINGS

Fig. 1 is the amino acid sequence of the SMN coding region of the clone T-BCD541.
Fig. 2A is the nucleotide sequence of the SMN coding region as well as the 5' and 3' flanking regions of clone C-BCD541 ; the coding region is underlined.
Fig. 2B contains the sequence starting from intron 6 up to exon 8 of the C-BCD541 gene. The underlined sequences are those of exons 7 and 8. Sequences of introns 6 and 7 can be chosen as oligonucleotides to amplify the cDNA region allowing the distinction, within exon 7, between the T-BCD541 gene and the C-BCD541 gene. The position of the divergent nucleotides between the T-BCD541 and C-BCD541 cDNA are in italics.
Fig. 3A is the nucleotide sequence of the SMN coding region as well as the 5' and 3' flanking regions of clone T-BCD541. The coding sequences are underlined. The numbers of the exons are indicated on the sequence. Asteriks indicate the beginning of each exon. The nucleotides which are indicated in italics are those which differ between the C-BCD541 and the T-BCD541 genes.
Fig. 3B represents the sequence from intron 6 up to the end of exon 8 of the T-BCD541 gene. The sequence of exons 7 and 8 is underlined.

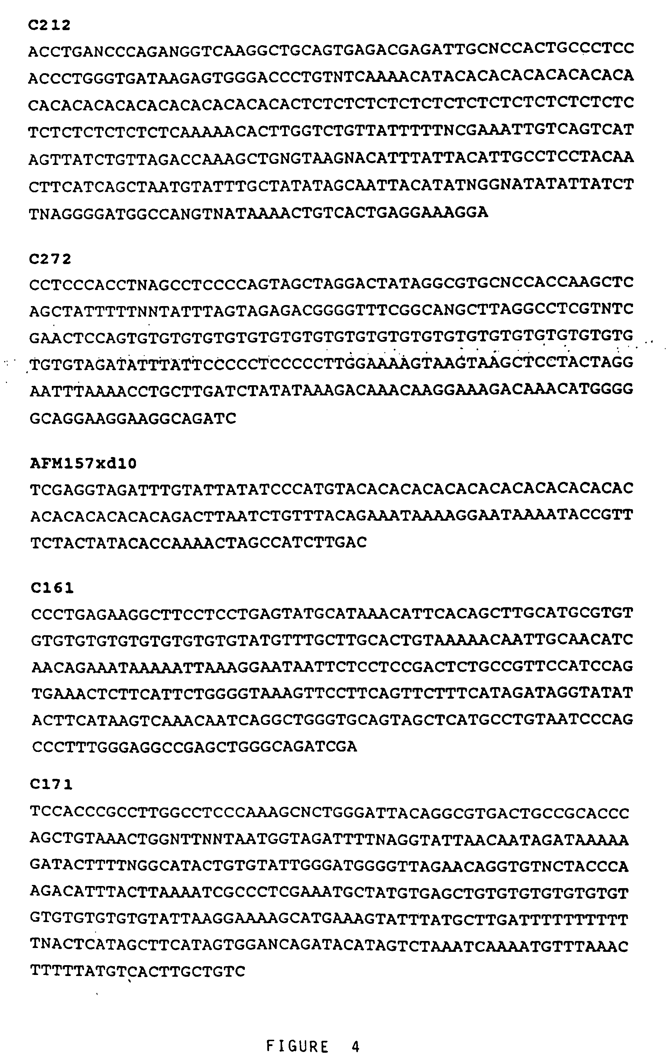

Fig. 4 is the nucleotide sequences of the markers C212, C272, C171, AFM157xd10, and C161.

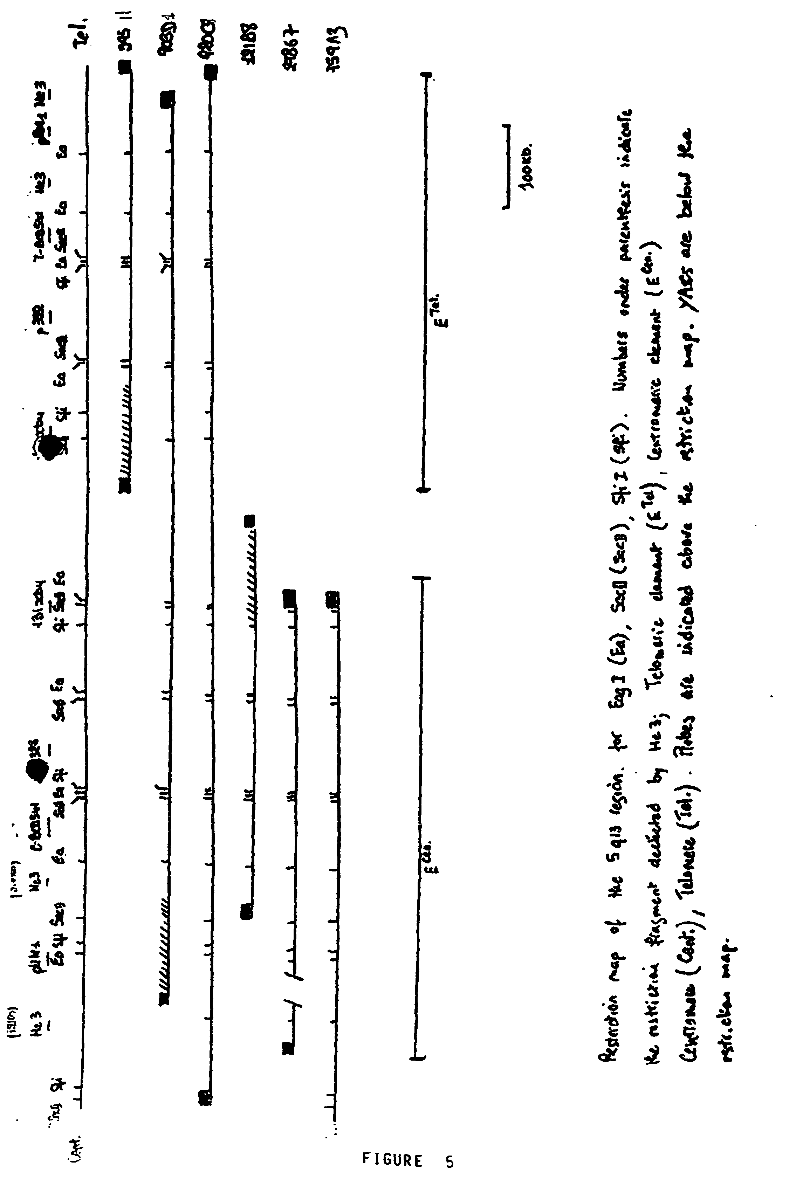

Fig. 5 represents various probes utilized in the present invention revealing several loci that the probes hybridized to.

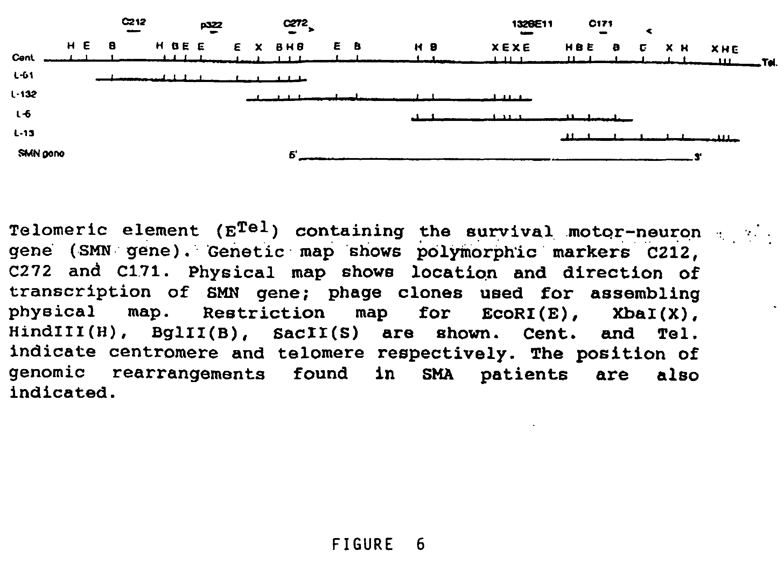

Fig. 6 represents the telomeric element containing the survival SMN gene.

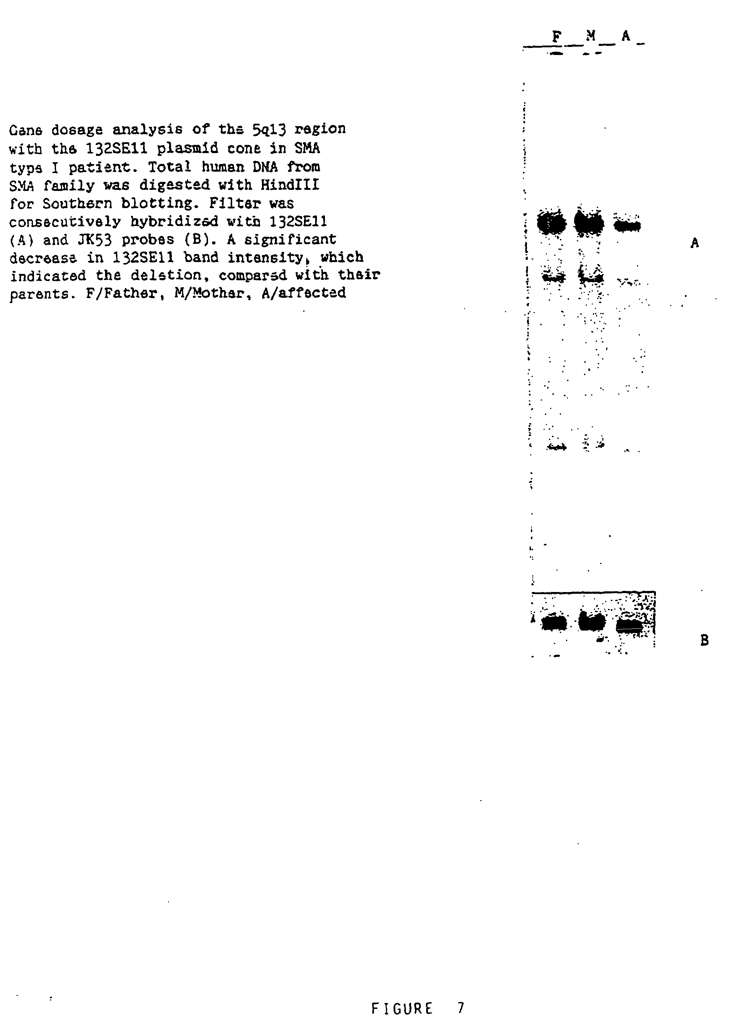

Fig. 7 represents the marked decrease of gene dosage with probe 132SEII, mapping close to this.
Fig. 8 represents the amino acid sequence of the truncated SMN protein.

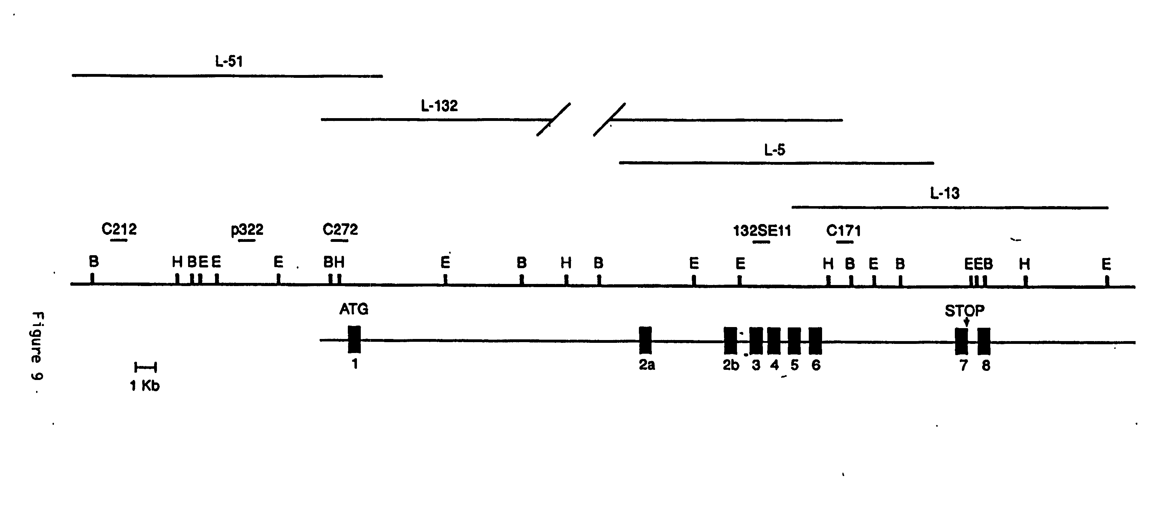

Fig. 9 is a schematic representation of the genomic structure of the human SMN gene. The designations and positions of genomic clones are shown above the figure. L-132, L-5, and L-13 depict the genomic clones spanning the entire SMN gene, while L-51 spans part of exon 1. Micro satellites and DNA markers are indicated above the genomic map. B, H, and E mean BglII, Hindlll and EcoRI, respectively. C212, p322, C272, 132SEII and C171 represent various markers. 1, 2a, 2b, 3, 4, 5, 6, 7, and 8 represent exons of the SMN and C-BCD541 genes. The entire sequence of L-132 is obtained by PCR amplification from exon 1 to exon 2A.
Fig. 10 represents the nucleotide sequence and amino acid sequence of the entire human SMN gene including the introns and exons. Translated nucleotide sequences are in upper case, with the corresponding amino acids shown below that. The polyadenylation signal is in bold face. Arrowheads indicate the position of the single base differences between SMN and C-BCD541 genes in introns 6 and 7 and exons 7 and 8. Italic letters indicate the position of the oligonulcoeitdes chosen for the detection of divergences in intron 7. (*) indicates the position of the stop codon.

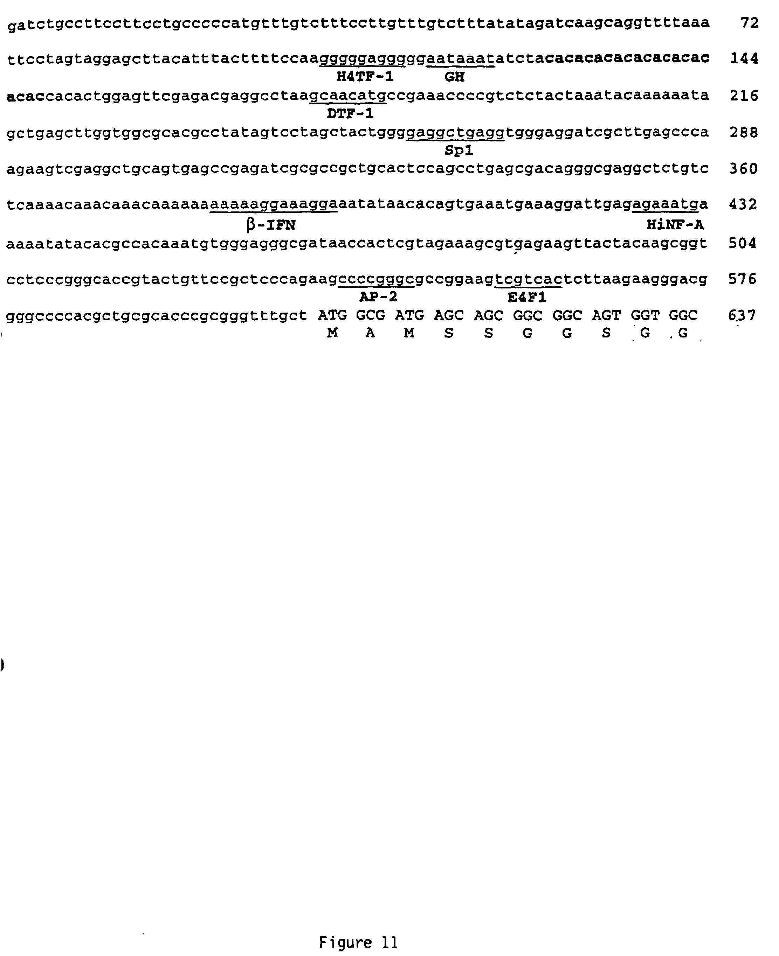

Fig. 11 represents the nucleotide sequence upstream of the coding region of the human SMN gene and illustrates the presence of putative binding sites for the transcription factors of AP-2, GH-CSE2, DTF-1, E4FI, HINF-A, H4TF-1, β-IFN and Spl. Bold letters indicate the dinucleotide repeat (CA) corresponding to the C272 markers.

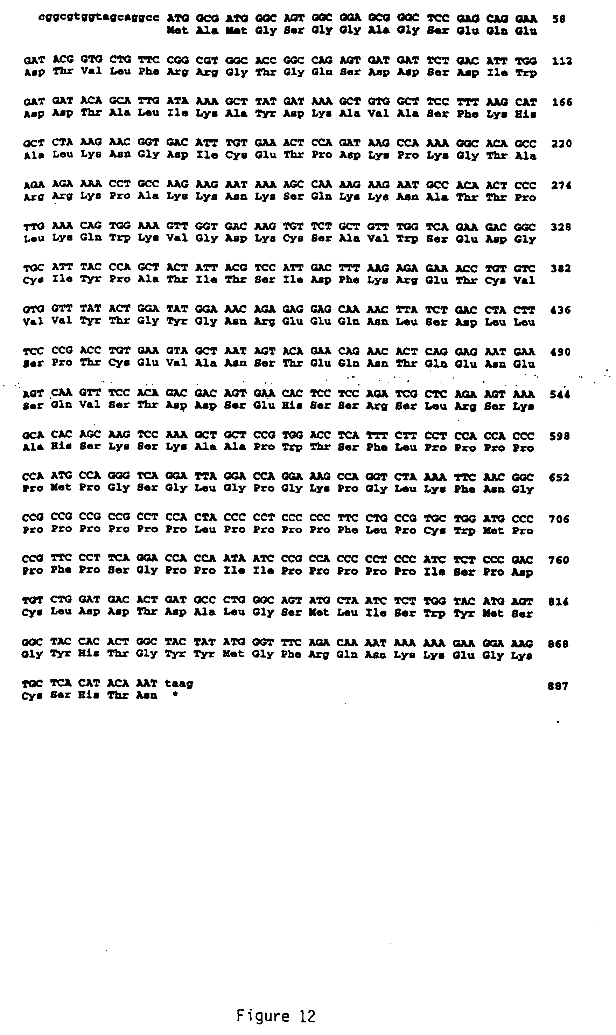

Fig. 12 represents the nucleotide and amino acid sequences of Mouse SMN cDNA. (*) indicates the position of the stop codon.

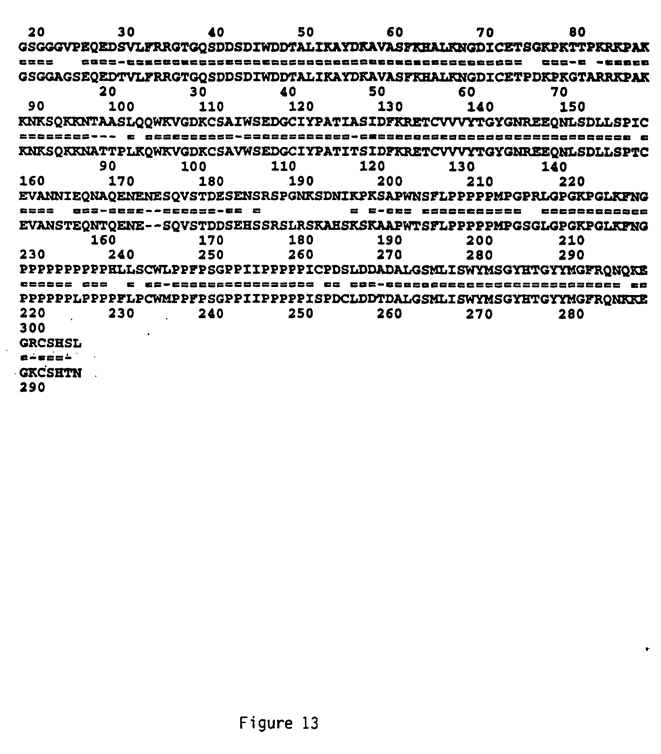

Fig. 13 represents a comparative analysis of the amino acid sequence of human SMN (above) and mouse SMN (below).

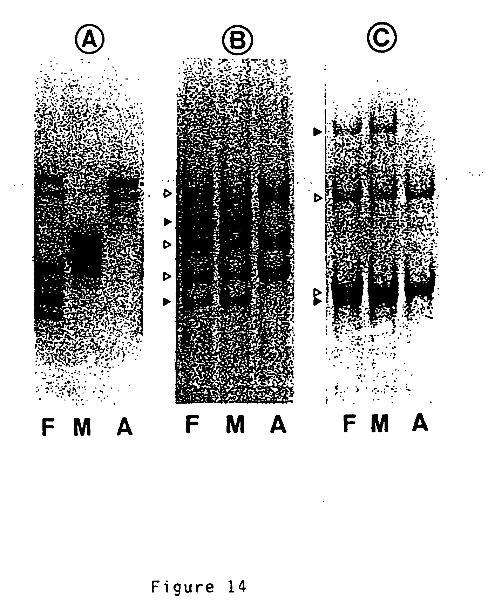

Fig. 14 illustrates the genetic analysis of family 6. Lane A shows evidence of inherited maternal deletion seen with the microsatellite marker C272 as the proband inherited only allele from the father. Lanes B and C represent SSCP analysis of PCR-amplified exons 7 (lane B) and 8 (lane C) of SMN (closed arrowheads) and its centromeric copy (open arrowheads). "F" represents the father, "M" the mother, and "A" the affected infant.

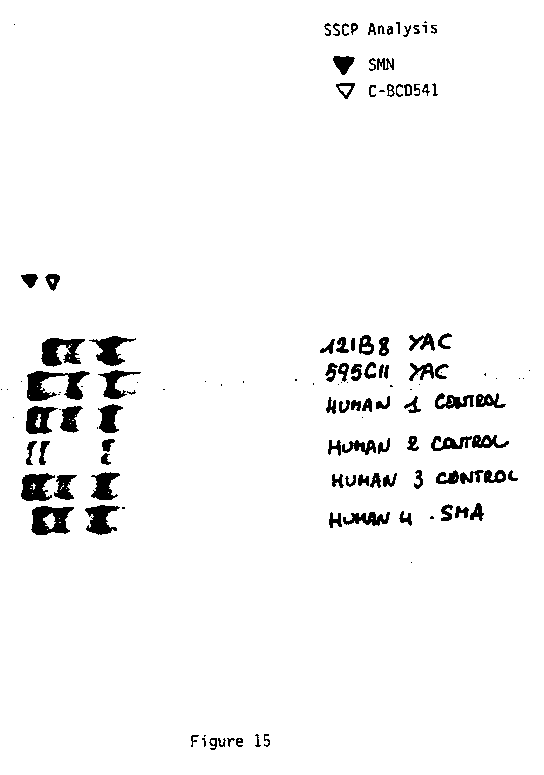

Fig. 15 illustrates the band shifts on single strand confirmation polymorphism (SSCP) analysis of the PCR amplified intron 7 and permitted indetification of SMN (closed arrowheads) and its centromeric counterpart C-BCD541 (open arrowheads).

### DETAILED DESCRIPTION OF THE PREFERRED EMBODIMENTS OF THE INVENTION

As used herein, the term "contig" means overlapping nucleotide sequences.

Previous studies by means of linkagee analysis have shown that all three forms of spinal muscular atrophy map to chromosome 5q11.2-q13.3. (L.M. Brzustowicz et al, Nature, **344**, 540 (1990) ; J. Melki et al, Nature, **345**, 823 (1990); J. Melki et al, Lancet, **336**, 271 (1990). A yeast artificial chromosome (YAC) contig of the 5q13 region spanning the disease locus was constructed that showed the presence of low copy-repeats in this region. Allele segregation was analyzed at the closest genetic loci detected by markers derived from the YAC contig (C212, C272 and C161) in 201 SMA families. These markers revealed two loci (C212, C272) or three loci on the 5q13 region (C161). Inherited and de novo deletions were observed in 9 unrelated SMA patients. Moreover, deletions were strongly suggested in at least 18% of SMA type I patients by the observation of marked heterozygosity deficiency for the loci studied. These results indicated that deletion events are statistically associated with the severe form of SMA.

By studying all polymorphic DNA markers derived from the YAC contig, it was observed that the smallest rearrangement occured within a region bordered by loci detected by C161 and C212-C272 and entirely contained in a 1.2-Mb YAC clone 903D1. See, for example, French Patent FR 2720757.

The present invention characterized the small nested critical SMA region of about 140 Kb by a combination of genetic and physical mapping in SMA patients. This region suggested a precise location for the SMA gene and therefore, a limited region within which to search for candidate genes. The present invention identified a duplicated gene from the 5q13 region. One of them (the telomeric gene) is localized within the critical region. Moreover, this gene was lacking in 213 out of 230 (92.2%) or interrupted in 13 out of 230 (5.6%) SMA patients. In patients where the telomeric gene is not lacking or interrupted, deleterious mutations indicated that this telomeric gene, termed survival motor-neuron (SMN) gene, is the chromosome 5 SMA-determining gene.

The SMN gene was discovered using a complex system of restriction mapping, distinguishing the E^{Tel} from the E^{Cen} by Southern blot, and the determination of the differences between the E^{Tel} in SMA patients by genetic and physical mapping. After confirming the location of the SMN gene, a phage contig spanning the critical region of the telomeric element was constructed to identify specific clones containing the SMN gene.

Analysis of the SMN gene in SMA patients compared with those of normal patients revealed either the SMN gene was either lacking or truncated in 98% of SMA patients or had combined mutations not present in normal control patients.

To identify a large inverted duplication and a complex genomic organisation of the 5q13 region, long-range restriction mapping using pulsed field gel electrophoresis (PFGE) of the YAC contig was performed.

YACs were ordered by comparing their haplotypes with that of the human donor at the polymorphic loci detected markers C212, C272, C171 and C161 (Fig. 4).

The restriction enzymes SacII, BssHII, Sfil, EagI and Xhol were used to digest the YACs containing the telomeric loci detected by markers C212, C272, C 171 and C161 (YAC clone 595C11), the centromeric loci detected by these markers (YAC clones 121 B8, 759A3, 278G7) or both (YAC clones 903D1 and 920C9). Lambda phage libraries of YACs 595C11, 121B8 and 903D1 were constructed and subclones from phages containing markers C212 (p322), C272 (132SE11), C161(He3), AFM157xd10(131xb4) and CMS1 (p11M1) were used as probes for PFGE analysis. Fig. 5 shows that probes 132SE11, 11P1 and p322 revealed two loci, and probe He3 revealed 4 loci on the YAC contig, whereas probe 131xb4 revealed several loci on 5p and 5q13. The restriction map (Figure 6) showed that the 5q13 region contained a large inverted duplication of an element (E) of at least 500 Kb, termed E^{Tel} and E^{Cen} for the telomeric and centromeric elements, respectively.

The PFGE analysis of SMA and control individuals revealed a high degree of variability of restriction fragments which hampered the distinghishment of E^{Tel} from the E^{Cen} and the recognition of abnormal restriction fragments in SMA patients.

In order to distinguish between the E^{Tel} and the E^{Cen}, a Southern blot analysis was then performed. The Southern blot was performed by the methods described in Sambrook et al, *supra*.

More specifically, DNA from YAC clones, controls and SMA patients was digested with restriction enzymes SacI, KpnI, MspI, PstI, Pvull, EcoRI, HindIII, BgIII and Xbal for Southern blotting and hybridized with clones 132SE11, 11p1, He3, 131xb4 and p322 as probes. None of the probes except one (He3) detected a difference between the two duplicated elements. Three HindIII restriction fragments of 12, 11 and 3.7 Kb were detected by probe He3. A 12 Kb HindIII restriction fragment was detected in YAC clones 754H5 and 759A3, indicating that this fragment corresponded to the most centromeric locus in the E^{Cen}. Conversely, a 11 Kb Hindlll fragment was detected in YACs clones 595C11, 903D1 and 920C9 indicating that this fragment corresponded to a single locus on the E^{Tel}. Finally, a 3.7 Kb HindIII fragment was noted in non-overlapping YACs containing either E^{Tel} or E^{Cen}, indicating that this fragment corresponded to two different loci. Similar results were obtained with SacI and KpnI. The three restriction fragments detected by He3 were observed on the monochromosomal hybrid HHW105 (Carlock, L.R. et al, Am. J. of Human Genet., 1985, Vol. 37, p. 839) and in 30 unrelated, healthy individuals, confirming that these fragments were not due to polymorphisms. The Southern analysis results allowed one to distinguish E^{Tel} from the E^{Cen} in both controls and SMA patients.

Thus, once the E^{Tel} from the E^{Cen} was distinguished, it was necessary to determine the differences between the E^{Tel} in SMA patients and those of the normal control. This was done by using genetic and physical mapping. This genetic and physical mapping identified genomic rearrangements in the telomeric element of E^{Tel} of SMA patients.

It was previously shown that 9 out of 201 (9/201) SMA patients displayed large-scale deletions encompassing either one or the two loci detected by markers C212 and C272 on one mutant chromosome (J. Melki et al, Science, **264**, 1474 (1994)). On the other hand, 22 out of 30 (22/30) patients born to consanguineous parents including 13 out of 14 (13/14) type I and 9 out of 10 (9/10) type III SMA, were homozygous by descent for the most closely flanking polymorphic markers.

The genomic DNA of the 9 patients harboring large scale deletions and the 22 consanguineous patients displaying homozygosity by descent were digested with HindIII for Southern blotting and hybridized with probe He3. The 11 Kb fragment revealed by probe He3 was absent in 12 out of 13 (12/13) consanguineous type I patients. In 2 out of 12 (2/12), the deletion also involved the 3.7 Kb fragment. By contrast, the 11 Kb fragment was absent in 1 out of 8 (1/8) consanguineous type III patients only. Consistently, the 11 Kb Hindlll fragment was absent in 4 out of 9 (4/9) patients harboring large scale deletions on one mutant chromosome. Of particular interest was the absence of the 11 Kb fragment in the patient harboring a deletion of one of the two loci detected by markers C212 and C272.

When analyzed together, these observations provided evidence for genomic rearrangements of E^{Tel} in SMA patients and supported the location of the SMA gene centromeric to the locus revealed by the 11 Kb Hindlll fragment, since all consanguineous type III patients but one were not deleted for this locus.

In order to characterize the centromeric boundary of the genomic rearrangement in the disease, the allele segregation at loci detected by marker C272 in consanguineous SMA patients was analyzed. All consanguineous SMA type I patients had one single PCR amplification product, compared with 0 out of 60 controls. This marked heterozygosity deficiency was due to deletion of one of the two loci detected by C272, as indicated by the marked decrease of gene dosage with probe 132SE11, mapping close to this marker. By contrast, 7 out of 9 (7/9) consanguineous type III SMA patients had two C272 amplification products inherited from both parents, indicating homozygosity at each locus detected by marker C272. Moreover, no gene dosage effect was observed with probe 132SE11 indicating the absence of deletion involving the locus detected by C272 in type III consanguineous patients.

Assuming that the same locus is involved in all three types of SMA, these results indicate that the disease causing gene is distal to the telomeric locus detected by C272.

These studies place the SMA gene within the telomeric element E^{Tel}, between the telomeric loci detected by markers C272 and He3 (11 kb HindIII fragment). Based on long-range restriction mapping using PGFE of the YAC contig, this critical region is entirely contained in a 140 Kb SacII fragment of YAC clone 903D1 (or 150 Kb SacII fragment of YAC clone 920D9).

After confirming that the SMN gene was located on a 140 Kb SacII fragment a phage contig spanning the critical region of the telomeric element was constructed in order to identify and characterize the SMN gene.

Phage clones containing markers C212, C272, C171 and C161 were isolated from the λ phage libraries constructed from YAC clones 595C11 and 903D1 and used as a starting point for bidirectional walking. A phage contig (60 Kb) surrounding markers C212, C272 and C171 was constructed based on the restriction map of the phage clones (Fig. 6). To identify genes in the contig, the following three stategies were used :
1) a search for interspecies-conserved sequences was conducted ;
2) exon trapping method was performed ; and
3) direct cDNA selection was performed. The genomic probe 132SE11, derived from the phage containing the marker C272, gave positive hybridization signals with hamster DNA indicating the presence of interspecies-conserved sequences. The screening of a λgt10 human fetal brain cDNA library with probe 132SE11 resulted in the selection of 7 overlapping λ clones spanning 1.6 kbp. Sequence analysis of the clones revealed a 882 bp open-reading frame (ORF) and a 580 bp non-coding region. A 1.5 kbp clone (BCD541) contained the entire coding sequence and most of the 3' non-coding region. The 3' end of the cDNA along with its poly(A)⁺ tail was obtained by PCR-amplification of a lymphoblastoid cell line cDNA library.

Two cDNA clones lacked nucleotides 661 to 755, suggesting that an alternative splicing might have occured. Northern blot analysis of poly(A)⁺ RNA from various tissues including heart, brain, liver, muscle, lung, kidney and pancreas, revealed the presence of a widely expressed 1.7 kb transcript. The ORF encodes a putative protein of 294 amino acids with a predicted molecular weight of approximately 32 Kd.

A homology search using the FASTA and BLAST networks failed to detect any homology at either the nucleotide or the amino acid level.

To further distinguish whether there was any duplication of the BCD541 gene in the 5q13 region, BCD541 cDNA was used as a probe for Southern blot and PFGE analysis of YAC clones spanning the disease locus.

Specific hybridization with non-overlapping YACs containing either the E^{Cen} only (YAC clones 759A3, 121 B8 and 278G7), or containing the E^{Tel} only (YAC clone 595C11) provided evidence for duplication of the BCD541 gene. Each gene encompassed approximately 20 kb and displayed an identical restriction pattern. Evidence for head to head orientation of the two genes was derived from the location of the SacII and EagI restriction sites of the non-overlapping YAC clones containing either E^{Cen} or E^{Tel}, following hybridization experiments with probes BCD541 and p322 which flank the SacII and EagI sites of each element.

In order to look for divergences in the two copies of the BCD541 gene, the organization of the telomeric gene was characterized and compared to that of the centromeric counterpart. Genomic sequence analysis revealed that the telomeric BCD541 gene is composed of 8 exons (Fig. 3). However, it is now known that the previously known exon 2 is composed of 2 exons separated by an additional intron as set forth in Fig. 10, therefore the SMN gene is composed of 9 exons.

Starting from either the centromeric or telomeric gene loci (in YAC clones 121 B8 and 595C11, respectively), PCR-amplification and sequence of each exon and their flanking regions revealed five discrepancies between the centromeric and the telomeric BCD541 genes. The first one is a conservative substitution in exon 7 (codon 280) specific for the telomeric (TTC) or the centromeric BCD541 gene (TTT). The second one, located in the 3' non-coding region (exon 8 nucleotide n° 1155) is specific for the telomeric (TGG) or the centromeric BCD541 gene (TGA). Three other single base substitutions were observed in the sixth and seventh introns.

The observation of both versions of each exon (exon 7 and 8) on either YAC clones containing both gene loci (YAC clone 920C9) or the monochromosomal hybrid HHW105 demonstrated that these substitutions are neither allelic nor due to polymorphisms. Band shifts on SSCP analysis of amplified exons 7 and 8 allowed an easy distinction of the telomeric (T-BCD541) and centromeric genes (C-BCD541) in both controls and SMA patients. All the unrelated healthy controls tested (n=75) harbored the T-BCD541 gene as determined by SSCP analysis of exons 7 and 8 (100%). Most of them (89.3%) also harbored the C-BCD541 gene but 8 out of 75 (8/75) (10.7%) lacked the C-BCD541.

A total of 230 SMA patients were tested for single base substitutions detected in exons 7 and 8 by SSCP method after PCR-amplification of genomic DNA. Among them, 103 belonged to type I, 91 to type II, and 36 to type III. Interestingly, 213 out of 230 SMA patients (92.6%) lacked the T-BCD541 gene on both mutant chromosomes compared with 0 out of 75 controls (0%). Moreover, 13 out of 230 SMA patients (5.6%) lacked the T-BCD541 gene for exon 7 on both mutant chromosomes but retained the T-BCD541 gene for exon 8 compared with 0 out of 75 controls (0%). Finally, only 4 out of 230 SMA patients (1.7%) harbored the T-BCD541 gene as determined by SSCP analysis of exons 7 and 8.

These results show that the T-BCD541 gene is either lacking or truncated in 98% of SMA patients. In addition, these data support the view that the disease gene is located between the telomeric locus detected by C272 and exon 8 of the T-BCD541 gene. Therefore, according to the overlapping restriction map of the phage contig, the critical region is entirely contained in 20 kb, suggesting that the telomeric BCD541 gene is the chromosome 5 SMA-determining gene.

In order to demonstrate that the T-BCD541 gene is responsible for SMA, point mutations in the 4 SMA patients in whom no rearrangement of the T-BCD541 gene had been observed were searched. Direct sequencing of PCR amplification products of each exon with their flanking regions was performed in the four patients.

A 7 bp deletion in the 3' splice acceptor site of intron 6 (polypyrimidine tract) was found in patient SA. Sequence analysis of exon 7 flanking the deleted intron, recognized the sequence specific for the T-BCD541 gene. Moreover, the non-deleted PCR-product corresponding to the same region, harbored the sequence specific for the C-BCD541 suggesting that the other mutant allele lacked the T-BCD541 gene.

In patient BI, a 4 bp deletion in the 5' consensus splice donor site of intron 7 was found. This deletion occured on the T-BCD541 gene as determined by sequence analysis of the flanking exon 7.

In patient HU, a point mutation in codon 272 (TAT→TGT) was found. This mutation changed a Tyrosine to Cysteine. The patient was heterozygous for the mutation, presumably carrying a different SMA mutation on the other allele. All three mutations observed in patients SA, HU and BI were not detected in 100 normal chromosomes ruling out rare polymorphisms.

A different splicing of exon 7 distinguished the C-BCD541 from the T-BCD541 gene using reverse transcription-based PCR. Eleven SMA patients were selected for the analysis of their transcripts by Northern blot or reverse transcription-based PCR amplification. Eight of them belonged to type I, 1 to type II and 2 to type III. SSCP analysis of genomic DNA showed an absence of T-BCD541 gene in 10 patients and one patient (SA) had C-BCD541 and T-BCD541 genes for both exons 7 and 8. Six unrelated controls who harbored both C-BCD541 and T-BCD541 genes and 2 controls with only T-BCD541 gene were included in the present study.

The expression of this gene in lymphoblasts made it possible to analyze the BCD541 transcripts in cell lines derived from controls and SMA patients. Northern blot analysis of RNA from lymphoblastoid cell lines showed the presence of a 1.7 kb mRNA in all samples. None of the SMA patients showed a transcript of altered size. It was observed that a reduced level of transcripts was obtained when compared to the expression of the β-actine gene in 3 out of 4 type I SMA patients. Normal mRNA level were found for the other SMA probands.

Since the Northern blot analysis revealed the presence of a transcript in SMA patients who had the C-BCD541 gene only for both exons 7 and 8 as determined by SSCP analysis, these results indicated that both C-BCD541 and T-BCD541 genes were expressed. To prove whether both BCD541 genes were expressed, RT-based PCR amplification of RNA isolated from the lymphoblastoid cell lines from controls and SMA patients was used. Direct sequencing of PCR products flanking exons 7 and 8 revealed that patients who had C-BCD541 only displayed the sequence specific for the C-BCD541 gene. Controls who had both T-BCD541 and C-BCD541 genes, had two types of transcripts corresponding to both BCD541 genes. These results confirmed that both genes were expressed. In addition, 2 alternative splicings involving exon 5 or exon 7 that resulted in different transcripts were observed. The alternative splicing of exon 5 confirmed previous sequence data on the cDNA clones.

The analysis of the RT-PCR amplification products encompassing exons 6 to 8 showed that the spliced transcript keeping exon 7, was present in controls who had both C-BCD541 and T-BCD541 genes or controls who had the T-BCD541 gene only. Conversely, the alternative spliced transcript lacking exon 7 was observed in controls who had both genes, but not in controls who had the T-BCD541 gene only. These results indicated that the alternative spliced transcript lacking exon 7 was derived from the C-BCD541 gene only.

The transcript analysis of patient SA harboring a 7 bp deletion of the 3' splice acceptor site of intron 6 of the T-BCD541 gene revealed the presence of both spliced transcript keeping exon 7 and alternate spliced transcript lacking exon 7. Moreover, the sequence analysis of amplification products from the spliced transcript keeping exon 7, showed a sequence specific for the C-BCD541 gene (Fig. 2). These results demonstrated that the 7 bp deletion of intron 6 observed in patient SA was deleterious for the correct splicing of exon 7 of T-BCD541 gene only. In addition, because a differential splicing of exon 7 allowed one to distinguish the 2 BCD541 genes, this difference was analyzed among controls and SMA patients including patient SA. In controls, the amount of alternated spliced transcript lacking exon 7 was less abundant than that of spliced product keeping exon 7. Conversely, in SMA patients, the amount of alternated spliced transcript lacking exon 7 was equal or more abundant than that of spliced product keeping exon 7.

These results provide evidence for a difference between controls and SMA patients at the transcription level of these genes. The alternative spliced transcript lacking exon 7 resulted in a shorter ORF with a different C-terminus protein that might have effects on the protein function.

To further characterize the entire structure and organization of the human SMN gene, three genomic clones were isolated from a FIX II phage library derived from YAC clone 595C11 and screened with the full-length BCD541 cDNA (Fig. 2A) as a probe. After selecting several clones that hybridized to the probe, restriction mapping and Southern blot analysis indicated that phages L-132, L-5 and L-13 spanned the entire SMN gene.

These three phage clones were further subjected to sequencing using the Maxam-Gilbert or Sanger et al methods of sequencing disclosed in Sambrook et al *supra*.

The nucleotide and amino acid sequence of the entire SMN gene including exons and introns is set forth in Figure 10. The human gene is approximately 20 kb in length and consists of nine (9) exons interrupted by 8 introns as shown in Figure 10. The human SMN gene has a molecular weight of approximately 32 kDA.

Although it was thought that only one exon 2 was present in the SMN gene (see, Lefebvre et al, Cell, 80:155-165 (1995)), the sequencing data proved otherwise and the previously mentioned exon 2 in Lefebvre et al *supra* is in fact composed of 2 exons separated by an additional intron, as illustrated in Figures 9 and 10. To avoid confusion in the renumbering of exons, the 2 exons in exon 2 are now referred to as exon 2a and exon 2b.

All exon-intron bounderies displayed the consensus sequence found in other human genes and a polyadenylation consensus site is localized 550 bp downstream from the stop codon (Fig. 10).

Starting from either YAC clones 121 B8 or 595C11 (which contain the C-BCD541 and SMN genes respectively, (see, Lefebvre et al, *supra*) PCR amplification and sequence analysis of the introns showed three differences between SMN and C-BCD541 in addition to those previously described (by Lefebvre et al, *supra*). These included a base change in intron 6 (-45bp/exon 7, atgt, telomeric; atat, centromeric) and two changes in intron 7 (+100bp/exon 7, ttaa, telomeric; ttag, centromeric and at position +214bp/exon 7, ttat, telomeric; ttgt, centromeric, Figure 10). The presence of both versions in a YAC clone containing both genes (YAC 920C9), and in the control population demonstrated that these substitutions are locus- specific rather than due to polymorphism. Band shifts on single strand conformation polymorphism (SSCP) analysis of the PCR amplified intron 7 allowed SMN and its centromeric counterpart (C-BCD541) to be readily distinguished (see, Figure 15).

In order to identify sequences potentially important for promoter function, the organization of the region surrounding exon 1 of the SMN and C-BCD541 genes was characterized. Based on restriction mapping, Southern blot hybridization and PCR amplification, exon 1 and the C272 marker (D5F150S1, D5F150S2) were located in the same BglII-EcoRI restriction fragment of L-132 phage (Figure 9). PCR amplification using the C272f primer and a reverse primer chosen in exon 1 was performed and the amplified product was directly sequenced. Sequence analysis showed that the (CA) repeat corresponding to the C272 marker are located 463bp upstream from the putative ATG translation start site (Figure 11). Comparative sequence analyses showed no discrepancy between the 5' ends of the SMN gene and its centromeric counterpart (C-BCD541). In addition, sequence analysis showed the presence of putative binding sites for the following transcription factors: AP-2, GH-CSE2, DTF-1, E4F1, HiNF-A, H4TF-1, β-IFN, Sp1 (Figure 11 ; Faisst et al, Nucleic Acids Res., 20:3-26 (1992)).

Besides isolating and characterizing the human SMN gene, the mouse homologue of the SMN gene was also cloned. Cross-species conservation of human SMN gene with rodents has been shown in Lefebvre et al, *supra* and served to isolate the mouse SMN gene. Screening of a mouse fetal cDNA library using human SMN cDNA as a probe allowed the isolation of 2 overlapping mouse cDNA clones. Sequence analysis of the clones revealed an 864 bp open-reading frame (ORF) (Fig. 12). The ORF encodes a putative protein of 288 amino acids (Fig. 12) with an homology of 83% with human SMN amino acid sequence (Fig 13).

Either the isolated human or the mouse SMN, the gene can be inserted into various plasmids such as pUC18, pBr322, pUC100, λgHI, λ18-23, λZAP, λORF8, and the like. The methods for inserting genes into different plasmid vectors are described by Sambrook et al *supra*. Various microorganisms can be used to transform the vector to produce the SMN gene. For example, host microorganisms include, but are not limited to, yeast, CHO cells, E. coli, Bacillus subtilis and the like.

Once recombinantly produced, the human SMN protein or the mouse SMN protein can be further purified from the host culture by methods known in the art.

Besides recombinantly producing the SMN protein, the present invention also relates to the production of polyclonal and monoclonal antibodies. These methods are known in the art as evidenced by Sambrook et al *supra*. The monoclonal antibody can be obtained by the procedure of Kohler and Milstein, Nature, 256:495 (1975) ; Eur. J. Immunol., 6:511 (1976) or Harlow and Lane Antibodies, a Laboratory Manual, Cold Spring Harbor Laboratory, Cold Spring Harbor, New York (1988), and can be used, for example, in diagnosing SMA, as well as other motor neuron disorders.

Polyclonal rabbit antisera can also be generated against synthetic peptides corresponding to any part of the SMN amino acid sequence including the amino terminus and carboxy terminus. More specifically, the following peptides were synthesized based on the amino acid sequence set forth in Figure 1 :

The synthetic peptide may be coupled to a carrier protein such as Keyhole limpet hemocyanin (KLH) through an amino- or carboxy-artificial cysteine residue that may be synthetically added to the desired sequence. The cysteine residue is used as a linker to couple the synthetic peptide to the carrier protein. The procedure utilized to couple synthetic peptides to KLH is described by Green et al, Cell, 28:477 (1982).

Approximately, 50-100 µg, preferably 100 µg of synthetic antigen is dissolved in buffer and emulsified with an equal volume of Freund's complete adjuvant. About .025 ml to 0.5 ml of emulsified antigen-adjuvant can be injected intramuscularly or intradermaly into a rabbit. Four to six weeks later, the rabbit is boosted and 20-40 ml of blood is drawn 7-10 days after each booster injection. The serum is then tested for the presence of antigen using RIA, ELISA or immunoprecipitation. The positive antibody fractions may then be purified, for example by absorption to protein A following the method of Goudswaald et al, Scand. J. Immunol., 8:21 (1978).

More specifically, about 20 to 50 µg of antigen, prepared either by the recombinant techniques set forth above or synthetically made antigen is diluted in about 100 µl of buffer and emulsified with an equal amount of Freund's complete adjuvant. About 30-60, preferably 50 µl of the emulsified antigen-adjuvant is injected subcutaneously at four sites into mice. Four to six weeks later, the mice are boosted with an intraperitoneal injection of about 100 µl containing 5-10 µg of antigen solubilized in buffer. The mice are bled from the mediam tail vein 7-10 days after the boaster injection and the serum is tested for antibody using standard methods. Blood is then drawn every 3-4 days until the antibody titer drops.

Tissue, plasma, serum, cerebral spinal fluid and the like can be used to detect SMA disease using the above-described monoclonal or polyclonal antibodies via Western blot (1 or 2 dimensional) or ELISA. These methods are known in the art as described by Sambrook et al, *supra*.

A method for detecting SMA as well as in ALS, ACM, and PLS patients who possibly have these motor neuron disorders, is also encompassed by the present invention. This method involves extracting from a patient suspected of having SMA, DNA from a sample. This sample may include sera, plasma, cerebral spinal fluid and the like. After extracting the DNA by known methods in the art, primers that are derived from exons 7 and 8 of the SMN gene are used to amplify the DNA.

After amplification with the primer, the amplified product is subjected to SSCP (Single Strand Conformation Polymorphism).

The gels are then subjected to autoradiography to determine if SMA is present in the sample.

More specifically, it has recently been discovered that in twelve cases of arthrogryposis multiplex congenita (AMC) associated with SMA, 6 out of 12 patients lacked the SMN gene.

A total of twelve unrelated patients including eight males and four females of various geographic origins was selected for the study. The patients were chosen based on the criteria that these patients had :
(1) congenital joint contractures of at least two regions of the body (see, Stern, JAMA, 81:1507-1510 (1923)) ;
(2) generalized muscle weakness with muscular atrophy and areflexia without extraocular involvement ;
(3) electromyographic studies showed denervation and diminished motor action potential amplitude ; and
(4) muscle biopsies consistent with denervation with no evidence of storage material or other structural abnormalities (see, Munsat, Neuromuscular Disorders, 1:81 (1991)).

The study consisted of Dinucleotide Repeat Polymorphism Analysis and SMN gene analysis (see, Examples) based on DNA extracted from peripheral blood leukocytes, lymlphoblastoid cell lines or muscle tissue in all twelve patients.

The data from this study is summarized in Table 1 below.

The diagnosis was made at birth with an uniform phenotype characterized by a severe hypotonia, absence of movements except extraocular mobility and contractures of at least two joints. The number of affected joints and the severity of the postural defects varied from infant to infant, as set forth in Table 1. Decreased fetal movements were noted in 7 out of 12 (7/12) patients. Neonatal respiratory distress was observed in 9 out of 12 (9/12) patients and facial involvement associated with micrognathia was noted in 4 out of 12 (4/12) patients. Most of the cases, 8 out of 12 (8/12), died within the first month of life. Four infants are still alive. No family history was noted except in family 12 in which both the child and her father were affected suggesting an autosomal dominant form of AMC.

Table 1 shows that the SMN gene was lacking on both mutant chromosomes in 6 out of 12 (6/12) patients (cases 1-6). Among them, 3 out of 6 (3/6) patients had a large inherited deletion involving both loci detected by markers C212 and C272 on one parental allele, the other parental carrying only one locus instead of the expected two, as shown in Figure 14.

Analysis of SMN exons did not reveal intragenic mutations in the patients whose SMN gene showed no deletions (cases 7-12). Genetic analysis showed that the disease gene in a family (case 9) was not linked to chromosome 5q13 as both the affected and healthy siblings carried the same 5q13 haplotype. These data strongly suggest that the patients whose SMN gene showed no deletions were not linked to the 5q13 SMA locus (cases 7-12).

Hitherto, arthrogryposis was regarded as an exclusion criterion in SMA (see, Munsat, *supra*). But the observation of SMN gene deletion in 6 out of 12 (6/12) patients (50%) strongly indicates that arthrogryposis of neurogenic origin is related to SMA and that this subgroup and SMA are allelic disorders. Yet, AMC of neurogenic origin is a genetically heterogeneous condition since the disease gene was not linked to SMN locus in 6 out of 12 (6/12) patients. Exclusion of chromosome 5q has also been shown in one family with two AMC-SMA patients, as described by Lunt et al, J. Med. Genet., 29:273 (Abstract) (1992).

Thus, by dinucleotide Repeat Polymorphism Analysis and SMN gene analysis, clinical diagnosis of AMC can be confirmed by the absence or interruption of the SMN gene. The present invention now provides methods to detect AMC either in live patients or in utero.

Yet another embodiment of the present invention is the detection of SMA using specific oligonucleotide probes based on the nucleotide sequence set forth in Figures 3, 10, or for the mouse SMA Figure 12. If a patient totally is lacking in the SMN gene, no hybridization to the specific probe will occur. The hybridization conditions may vary depending upon the type of sample utilized. It is preferable to conduct such hybridization analysis under stringent conditions which are known in the art and defined in Sambrook et al *supra*. The oligonucleotide probes may be labeled in any manner such as with enzymes, radioactivity and the like. It is preferable to use radiolabeled probes.

In another embodiment of the present invention, the human SMN gene can be utilized in conjunction with a viral or non-viral vector for administration in vivo directly to the patients suffering from SMA or related motor neuron diseases or by administration in vitro in bone marrow cells, epithelial cells fibroplasts, followed by administration to the patient. See, for example Resenfeld et al, Science (1991) 252, pp. 431 to 434.

The present invention provides a method of detecting SMN gene defects or the total lack of the SMN gene in a fetus. Amniotic fluid taken from the pregnant woman is subjected to SSCP analysis according to the methods of the present invention.

In order to further illustrate the present invention and advantages thereof, the following specific examples are given, it being understood that the same are intended only as illustration and in nowise limitative.

### EXAMPLES

### EXAMPLE 1

### Construction of phage libraries from the 121 B8, 595C11, and 903D1 YAC clone.

Total yeast DNA from YAC clone 595C11 contaiinng the telomeric loci detected by C212, C272 and C161, or YAC clone 121B8 containing the centromeric loci detected by the same markers or 903D1 YAC clone containing both loci was purified and partially digested with Sau3A. DNA in the size range of 12 to 23 kb was excised after 0.5% Seaplaque GTG agarose gel electrophoresis and precipitated with ethanol after β-agarase digestion. After partial fill-in of the Sau3A site, DNA was subcloned at the partially filled Xhol site of bacteriophage FIXIII (Stratagene). Clones of λ containing the microsatellite DNA markers C212 (L-51), C272 (L-51, L-132), C171 (L-5, L-13), C161 (595B1), 11M1 (L-11), AFM157xd10 (L-131) were digested either with EcoRI or HindIII or both and subcloned into pUC18 plasmid vectors. Subclones from phages containing markers C212(p322), C272(132SE11), C161(He3), AFM157xd10(131xb4) and CMS1(p11M1) were used as probes.

### EXAMPLE 2

### Pulsed field gel electrophoresis analysis

High molecular weight DNA was isolated in agarose plugs from Epstein-Barr virus transformed lymphoblastoid cell lines established from controls and patients or from YAC clone as described. Plugs were rinsed twice for 30 min. each in 10-20 min vol. TE. The plugs were equilibrated for 30' at 4°C with 0.3 ml of the appropriate restriction enzyme buffer containing 0.1 mg/ml BSA (Pharmacia). Excess buffer was then removed and the plugs were incubated at the appropriate temperature for 16 h with 40 U restriction enzyme per reaction. DNA was digested with the restriction enzymes BssHII, EagI, Sfil, SacI, KpnI, SacII, SpeI. Separation of DNA fragments was performed using a CHEF-III-DR PFGE apparatus (Biorad). Fragments from 50 to 1200 kb were separated by electrophoresis through 1% agarose Seakem, at 200 V for 24 h at 14°C in 0.5 XTBE running buffer using a 30' to 70' ramping pulse time. The separation of fragments from 5 to 100 kb was performed by electrophoresis at 200 V for 19 h at 14°C in 0.5 x TBE buffer using a 5' to 20' ramping pulse time. After treatment with 0.25N HCl for 20 min, pulsed field gels were blotted onto Hybond N+ Nylon membrane (Amersham) in 0.4N NaOH, 0.4M NaCl for 20 h. Probes were successively hybridized to the same filters to ensure accurate data. Hybridizations were performed as described.

### EXAMPLE 3

### YAC library screening

YAC libraries from CEPH were screened by PCR with microsatellites C212, C272, C171, CMS1, and C161. YAC genotypes were established by electrophoresis of PCR products on denaturing polyacrylamide gels. YAC size was estimated by pulsed field gel electrophoresis.

### EXAMPLE 4

### Southern blot analysis

DNA samples were extracted from either peripheral blood leukocytes or lymphoblastoid cell lines. DNA were digested with restriction enzymes EcoRI, Hindlll, BglII, Xbal, Pvull, Xmnl, Rsal, PstI, BamHI, separated by electrophoresis on an 0.8% agarose gel for Southern blotting and hybridized with radioactively labeled probes.

### EXAMPLE 5

### Dinucleotide repeat polymorphisms

Genotypic data were obtained for the C212(D5F149S1, -S2), C272(D5F150S1, -S2) and C161(D5F153S1, -S2) dinucleotide repeat. Amplification conditions were as follows : denaturation at 94°C, annealing at 55°C, and extension at 72°C, 1 min each for 30 cycles. The procedure used for detection of dinucleotide repeat polymorphisms has been described elsewhere.

### EXAMPLE 6

### cDNA clone and DNA sequencing

Two million recombinants of a λgt10 human fetal brain library were plated according to the manufacturer (Clontech). Prehybridization and hybridization was carried out in 10% Dextran Sulphate. Sodium, 1 M NaCl, 0.05 M Tris-HCl pH 7.5, 0.005 M EDTA and 1% SDS with 200 mg/ml sheared human placental DNA (Sigma) for 16 hours at 65°C. The filters were washed in 0.1X SSEP-0.1% SDS at 65°C and autoradiographs were performed for 24 hours. The DNA of positive cDNA clones were purified, digested with EcoRI and subcloned in M13 bacteriophage. Single strand DNAs were sequenced using the DyeDeoxy™ Terminator Cycle Sequencing Kit protocol supplied by Applied Biosystems, Inc. and analyzed on a ABI model 373A DNA automated sequencer. To obtain the 3' end of the cDNA along with its poly(A)⁺ tail, PCR-amplification of a lymphoblastoid cell line cDNA library was performed using specific primer complementary to the 3' end of the clones and primer specific to the vectors arms of the cDNA library as previously described (Fournier B., Saudubray J.M., Benichou B. et al, 1994, J. Clin. Invest. 94:526-531). Specific PCR-products were directly sequenced with both primers using the DyeDeoxy™ Terminator Cycle Sequencing Kit protocol supplied by Applied Biosystems, Inc. and analyzed on a ABI model 373A DNA automated sequencer.

### EXAMPLE 7

### Isolation of RNA and Northern blot analysis

mRNA from lymphoblast cell lines of controls and SMA patients were isolated with the QuickPrep mRNA purification kit (Pharmacia) according to the supplier's procedure. Total RNA was prepared following the single-step RNA isolation method described by Chomczynski and Sacchi (Analytic Biochemistry, 162:156-159 (1987)). The total RNA preparation was treated with RQ1-DNAse (Promega) to remove any contaminating genomic DNA. Northern blots were made from mRNA and total RNA by electrophoresis through 1.5% seakem agarose gel containing methyl mercuric hydroxide and transferred to positively charged membrane in 20 X SSC and heated for 2 hours at 80°C. ³²P-radiolabeled DNA probes were synthesized by a random priming method according to the manufacturer (Boehringer), and hybridized in a solution containing 5 X SSEP, 1% SDS, 5 X Denhardt's for 16 hours at 65°C. The membranes were washed to a final stringency of 0.1 X SSEP, 0.1% SDS at 65°C for 10 min. Autoradiography was at -80°C with intensifying screens and Kodak XAR films for 2 to 10 days. The amount of mRNA was normalized with a b-actine cDNA probe. The autoradiographs were scanned at 600 nm in computerized densitometer (Hoeffer Scientific Instruments, San Francisco). A Northern blot with polyA+ RNA from several huma tissues was purchased from Clontech.

### EXAMPLE 8

### Reverse transcriptase-based PCR amplification and sequencing

Each PCR amplification was carried out in a final volume of 20 ml on single-strand cDNAs synthesized from the random hexamers-primed reverse transcription (Promega). The PCR reactions included 2 picomoles of forward and reverse primers and 1 unit Taq polymerase in the reaction buffer recommended by Perkin Elmer/Cetus. Parameters for PCR amplification consisted in 1 min at 94°C, 1 min at 55°C and 1 min at 72°C for 30 cycles followed by a final extension period of 10 min at 72°C. Parameters for PCR amplification consisted in 1 min at 94°C, 1 min at 55°C and 1 min at 72°C for 30 cycles followed by a final extension period of 10 min at 72°C. The PCR products were cut from acrylamide gel and eluted in 100 ml of TE buffer. The diluted fragments were reamplified with the same primers prior direct sequencing. The PCR amplification products were cut from acrylamide gel and eluted in 100 ml of TE buffer. The diluted fragments were reamplified prior to direct sequencing with both primers using the DyeDeoxy™ Terminator Cycle Sequencing Kit protocol supplied by Applied Biosystems, Inc. and analyzed on a ABI model 373A DNA automated sequencer. Six sets of primers along the cDNA sequence were used to amplify DNA products for sequence analysis.

### EXAMPLE 9

### Computer-assisted Analysis

Sequence homology analysis with both nucleotide and protein sequences from 541C were performed using FASTA and BLAST through the CITI2 French network (Dessen P., Fondrat C., Velencien C., Mugnoer C., 1990, CABIOS; 6:355-356).

### EXAMPLE 10

### SSCP Analysis

For single strand conformation polymorphism (SSCP) analysis, DNA from peripheral leukocytes (200 ng) was submitted to PCR amplification using unlabelled primers (20 µM) in 25 µl amplification mixture containing 200 µM dNTPs, 1 unit of Taq polymerase (Gibco-BRL) and 0,1 µl of a ³²P dCTP (10mCi/ml, NEN). Amplified DNA was mixed with an equal volume of formamide loaded dye (95% formamide, 20 mM EDTA, 0.05% bromophenol blue, 0.05% xylene cyanol). The samples (5µl) were denatured for 10 mn at 95°C and loaded onto a polyacrylamide gel (Hydroling MED, Bioprobe) and electrophoresed at 4°C for 18 to 24 hours at 4W. Gels were transferred onto 3 MM Whatman paper, dried and autoradiographed with Kodak X-OMAT films for 24 hours. To amplify the DNA sequence containing the divergence of exon 7 oligonucleotides R111 (5' AGACTATCAACTTAATTTCTGATCA 3') and 541 C770 (5'°°TAAGGAATGTGAGCACCTTCCTTC 3') were used. To amplify the DNA sequence containing the divergence of exon 8 oligonucleotides 541C960 (5' GTAATAACCAAATGCAATGTGAA 3') and 541C1120 (5' CTACAACACCCTTCTCACAG 3') were used.

### EXAMPLE 11

### Cloning of the human SMN gene

Total yeast DNA from YAC clone 595C11 was purified via the method of Sambrook et al *supra* and partially digested with restriction enzyme Sau3A. DNA in the 12-23 kD size range was excised after 0.5% sea plague GTG agarose gel electrophoresis and precipitated with ethanol after β-agarase digestion. After partial fill-in of the Sau3A site, DNA was subcloned at the partially filled XhoI site of bacteriophage FIXII (Stratagene).

The full-length BCD541 cDNA was used as a probe to screen the FIXII phage library under conditions set forth in Sambrook et al, *supra*.

These phages, named M-132, L-5 and L-13 spanned the entire SMN gene as confirmed by restriction mapping using Hindlll, EcoRI and BglII (see, Fig. 9) and Southern blot analysis.

The phages were then sequenced as described in Example 8. Once the gene was sequenced, it was then cloned into a pUC18 vector and recombinantly reproduced in large quantities that were purified for further use.

### EXAMPLE 12

### Cloning of the mouse SMN gene

A mouse fetal cDNA library was screened using the coding sequence of the human SMN cDNA as a probe according to Sambrook et al, *supra*.

Two overlapping mouse cDNA clones were found that had the entire sequence of mouse SMN, as revealed by sequencing methods described in Example 8 after being cloned into a pUC18 vector and M13 vectors.

### EXAMPLE 13

### Transgenic mouse

Transgenic mice containing multiple normal SMN genes OR SMN genes lacking exon 7 are produced by the methods according to Lee et al, Neuron, 13; 978-988 (1994). The transgenic animals are then tested and selected for the overexpression of the SMN gene or SMN gene lacking exon 7 via Southern, and/or Northern blots using the probes described in the present invention or by screening with antibodies described in the present invention in a Western blot.

Transgenic mice containing abnormal SMN genes are obtained by homologous recombination methods using mutated SMN genes as described by Kühn et al, Science, 269; 1427-1429 (1995) and Bradley, Current Opinion in Biotechnology, 2; 823-829 (1991). The transgenic animals are then tested and selected for the overexpression of the SMN gene via Southern, and/or Northern blots using the probes described in the present invention or by screening with antibodies described in the present invention in a Western blot selected for the abnormal SMN gene.

### EXAMPLE 14

### Polyclonal antibodies

100 µg of a synthetic antigen having sequence : was dissolved in buffer and emulsified with an equal volume of Freund's complete adjuvant. 0.5 ml of the emulsified synthetic antigen-adjuvant was injected intramuscularly into a rabbit. Five weeks later, the rabbit was boosted and 20-40 ml of blood was drawn 8 days after each booster injection. The serum was then tested for the presence of antigen using RIA.

Polyclonal antibodies were also prepared by the same methods using the following sunthetic antigens :

### EXAMPLE 15

### Gene Therapy

Using the adenovirus construct described by Ragot et al, Nature, Vol. 361 (1993), the normal SMN gene was inserted therein and injected intramuscularly into a patient lacking this gene. The patient is monitored using SSCP analysis as described in Example 10 above.

It is intended that the scope of the present invention be limited solely by the scope of the following claims.

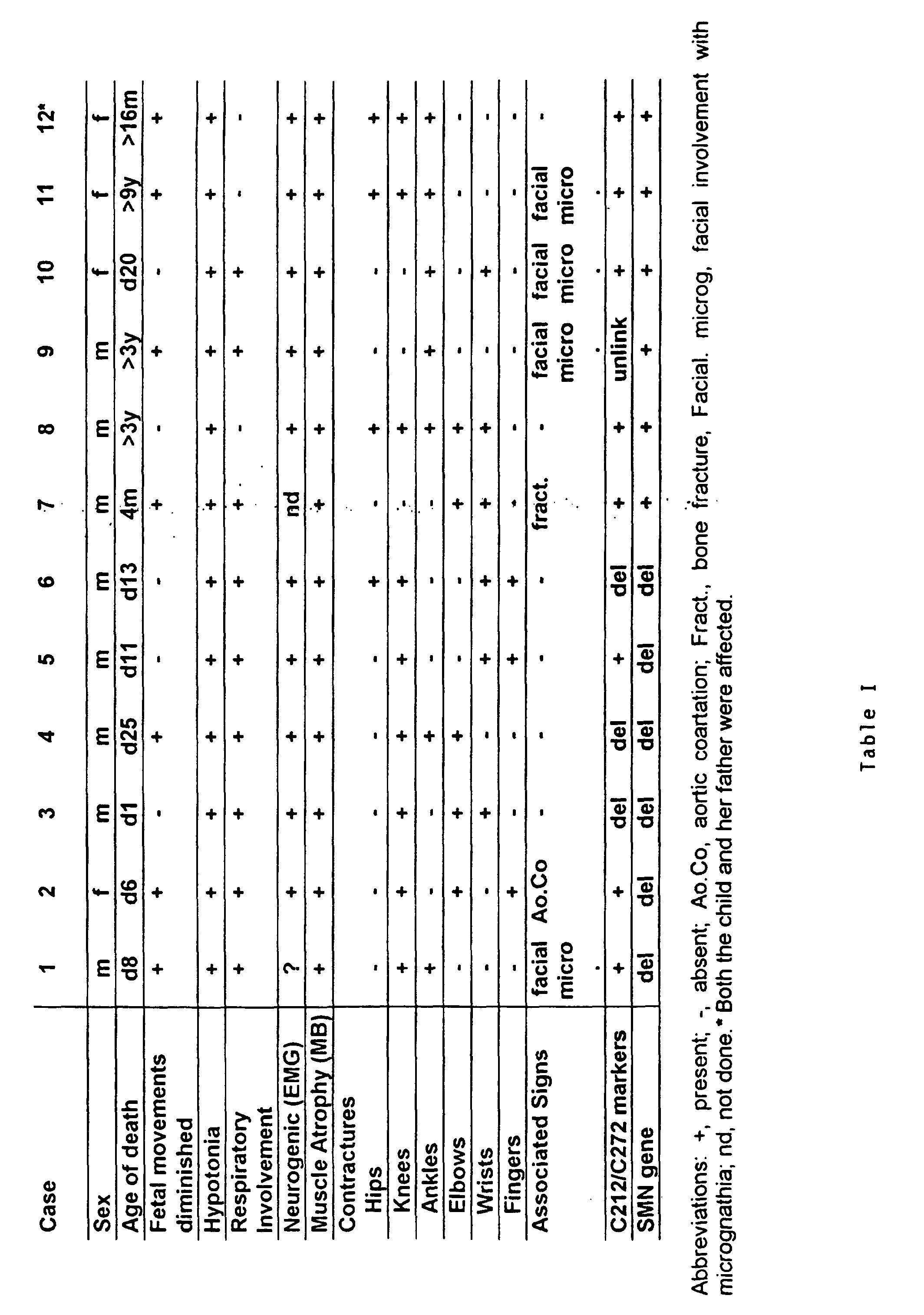

## Claims

1. An isolated human SMN gene T-BCD541 having the cDNA sequence which is underlined in Figure 3A (nucleotides 34 to 915).

2. The isolated SMN gene according to claim 1, which comprises intronic sequences having the following sequences:
- for intron n°6:
- for intion n°7:

3. An isolated variant of the SMN gene, which variant is a C-BCD541 gene having the cDNA sequence which is underlined in Figure 2A (nucleotides 34 to 915).

4. An isolated nucleotide sequence which is the cDNA sequence of a SMN gene according to claim 1 or claim 2, which cDNA has the sequence which is underlined in Figure 3A (nucleotides 34 to 915).

5. A coding sequence of the SMN gene according to claim 1 or claim 2, which sequence comprises nucleotides 34 to 915 of the sequence of Figure 3A.

6. A coding sequence of the variant of the SMN gene according to claim 3, which sequence comprises nucleotides 34 to 915 of the sequence of Figure 2A.

7. Intronic sequence of the SMN gene according to claim 1 or claim 2, which sequence has one of the following sequences:
- Intron 6:
- Intron 7:

8. An isolated DNA sequence encoding a survival motor neuron (SMN) protein of Figure 1 or Figure 8.

9. An isolated nucleotide sequence of Figure 11.

10. Oligonucleotide selected among the following sequences:

11. Set of oligonucleotides comprising a first oligonucleotide, comprising at least 9 nucleotides, which is comprised within a sequence of any one of claims 5 to 7, with the proviso that the first oligonucleotide does not contain the sequence p(dT)₈dAdA or p(dT)₈dA and a second oligonucleotide, comprising at least 9 nucleotides, which is comprised within the sequence selected from the group of : nucleotides 921 to 1469 of the sequence of Figure 3A, intron 7 having the following sequence: and the nucleotide sequence of Exon 7 in Figure 3A.

12. Set of primers comprising:
- a pair of primers having the following sequences:
- a pair of primers having the following sequences:
- a pair of primers having one of the following sequences:

13. Antisense nucleotide sequence which is an invert complementary sequence of a sequence according to any of claims 5 to 7.

14. An isolated human survival motor neuron (SMN) protein comprising the amino acid sequence of Figure 1.

15. A protein according to claim 13, which is truncated and which comprises the sequence of Figure 8.

16. Kit for the in vitro detection of a truncation, a deletion or a mutation of a survival motor neuron gene (SMN) indicative of spinal muscular atrophy or amyotrophic lateral sclerosis, comprising:
- a set of oligonucleotides according to claim 10 or claim 11;
- reagents for an amplification reaction; and
- a probe for the detection of the amplified product, wherein said probe is a fragment of a sequence of any one of claims 5 to 7, said fragment comprising at least 9 nucleotides.

17. Cloning or expression vector, **characterized in that** it comprises a sequence according to any one of claims 1 to 8.

18. Vector according to claim 17, **characterized in that** it is a poliovirus, an adenovirus or a herpes virus.

19. Vector according to claim 17, **characterized in that** it is a retrovirus vector.

20. An isolated Host cell, for example bone marrow cells, fibroblasts, epithelial cells, **characterized in that** it is transformed by a vector according to any one of claims 17 to 19.

21. An in vitro method for detecting the presence, absence or truncation of a survival motor neuron (SMN) gene indicative of a spinal muscular atrophy or amyotrophic lateral sclerosis, said method comprising the steps of:
(a) extracting DNA from a patient sample;
(b) amplifying said DNA with primers according to claim 11 or claim 12;
(c) subjecting said amplified DNA to Single Strand Conformation Polymorphism, wherein said analysis comprises comparing a pattern of DNA fragments obtained from a patient sample to a pattern of DNA samples obtained from a control sample to detect a truncation, a deletion or a mutation in the patient gene; and
(d) detecting the presence of a truncation, a deletion or a mutation in the patient gene in the survival motor neuron gene which is indicative of spinal muscular atrophy or amyotrophic lateral sclerosis.

22. The method of claim 21, wherein steps (c) and (d) are replaced with a step of digestion with a BsrI enzyme.

23. An in vitro method for detecting spinal muscular atrophy said method comprising the steps of :
(a) extracting DNA from a patient sample;
(b) hybridizing said DNA with a DNA probe comprising all or part of the DNA sequence of Figure 3 under stringent conditions; and
(c) detecting the hybrids possibly formed, wherein if no hybridization occurs, the patient sample lacks the survival motor neuron gene which is indicative of spinal muscular atrophy.

24. The method according to claim 23, wherein said probe is radiolabeled.

25. A monoclonal antibody or a polyclonal antiserum which specifically binds to the SMN protein of Figure 1, or the protein of Figure 8.

26. An in vitro method for detecting the absence or the interruption of a survival motor neuron gene indicative of arthrogryposis multiplex congenita (AMC), said method comprising the steps of:
(a) extracting DNA from a patient sample;
(b) amplifying said DNA via PCR using unlabeled primers from exon 7 and exon 8 of the SMN gene;
(c) subjecting said amplified DNA to Single Strand Conformation Polymorphism analysis, wherein said analysis comprises comparing a pattern of DNA fragments obtained from the patient sample to a pattern of DNA fragments from a control sample to detect the absence or interruption in the patient gene; and
(d) detecting the absence or the interruption of a survival motor neuron gene indicative of arthrogryposis multiplex congenita.

27. A transgenic mouse that expresses the human SMN protein of Figure 1 or truncated protein of Figure 8.

28. The transgenic mouse of claim 27, which is obtained by homologous recombination.

29. A transgenic mouse that expresses a mutated SMN protein of Figure 1.

## Patentansprüche

1. Isoliertes humanes SMN-Gen T-BCD541 mit der cDNA-Sequenz, die in der Figur 3A unterstrichen ist (Nukleotide 34 bis 915).

2. Isoliertes SMN-Gen gemäß Anspruch 1, das Intronsequenzen mit den folgenden Sequenzen umfasst:
- für Intron Nr. 6:
- für Intron Nr. 7:

3. Isolierte Variante des SMN-Gens, wobei die Variante ein C-BDC541-Gen mit der cDNA-Sequenz ist, die in der Figur 2A unterstrichen ist (Nukleotide 34 bis 915).

4. Isolierte Nukleotidsequenz, bei der es sich um die cDNA-Sequenz eines SMN-Gens gemäß Anspruch 1 oder 2 handelt, wobei die cDNA die Sequenz aufweist, die in der Figur 3A unterstrichen ist (Nukleotide 34 bis 915).

5. Codierende Sequenz des SMN-Gens gemäß Anspruch 1 oder 2, wobei die Sequenz die Nukleotide 34 bis 915 der Sequenz aus der Figur 3A umfasst.

6. Codierende Sequenz der Variante des SMN-Gens gemäß Anspruch 3, wobei die Sequenz die Nukleotide 34 bis 915 der Sequenz aus der Figur 2A umfasst.

7. Intronsequenz des SMN-Gens gemäß Anspruch 1 oder 2, wobei die Sequenz die folgenden Sequenzen aufweist:
- Intron 6:
- Intron 7:

8. Isolierte DNA-Sequenz, die für ein Protein für das Überleben der Motomeurone (survival motor neuron, SMN) gemäß Figur 1 oder Figur 8 codiert.

9. Isolierte Nukleotidsequenz gemäß Figur 11.

10. Oligonukleotid, ausgewählt aus den folgenden Sequenzen:

11. Set von Oligonukleotiden, umfassend ein erstes Oligonukleotid, enthaltend mindestens 9 Nukleotide, das innerhalb einer Sequenz gemäß einem der Ansprüche 5 bis 7 enthalten ist, mit der Maßgabe, dass das erste Oligonukleotid nicht die Sequenz p(dT)₈dAdA oder p(dT)₈dA enthält, und ein zweites Oligonukleotid, enthaltend mindestens 9 Nukleotide, das Innerhalb der Sequenz enthalten Ist, ausgewählt aus der Gruppe: Nukleotide 921 bis 1469 der Sequenz aus der Figur 3A, Intron 7 mit der folgenden Sequenz: und Nukleotidsequenz von Exon 7 in der Figur 3A.

12. Set von Primern umfassend:
- ein Paar von Primern mit den folgenden Sequenzen:
- ein Paar von Primern mit den folgenden Sequenzen:
- ein Paar von Primern mit den folgenden Sequenzen:

13. Antisense-Nukleotidsequenz, bei der es sich um eine invertierte komplementäre Sequenz einer Sequenz gemäß einem der Ansprüche 5 bis 7 handelt.

14. Isoliertes humanes Protein für das Überleben der Motomeurone (SMN), umfassend die Aminosäuresequenz aus der Figur 1.

15. Protein gemäß Anspruch 13, das trunkiert ist und das die Sequenz gemäß Figur 8 umfasst.

16. Kit für die in vitro Detektion einer Trunklerung, einer Deletion oder einer Mutation eines Gens für das Überleben der Motomeurone (SMN), das auf spinale Muskelatrophie oder amyotrophe Lateralsklerose hinweist, umfassend:
- ein Set von Oligonukleotiden gemäß Anspruch 10 oder Anspruch 11;
- Reagenzien für eine Amplifizierungsreaktion; und
- eine Sonde für die Detektion des amplifizierten Produkts, wobei es sich bei der Sonde um ein Fragment einer Sequenz gemäß einem der Ansprüche 5 bis 7 handelt, wobei das Fragment mindestens 9 Nukleotide umfasst.

17. Klonierungs- oder Expressionsvektor, **dadurch gekennzeichnet, dass** er eine Sequenz gemäß einem der Ansprüche 1 bis 8 umfasst.

18. Vektor gemäß Anspruch 17, **dadurch gekennzeichnet, dass** es sich um einen Pollovirus, einen Adenovirus oder einen Herpesvirus handelt.

19. Vektor gemäß Anspruch 17, **dadurch gekennzeichnet, dass** es sich um einen Retrovirus-Vektor handelt.

20. Isolierte Wirtszelle, beispielsweise Knochenmarkzellen, Fibroblasten, Epithelzellen, **dadurch gekennzeichnet, dass** sie mittels einem Vektor gemäß einem der Ansprüche 17 bis 19 transformiert ist.

21. In vitro-Methode zur Detektion des Vorliegens, der Abwesenheit oder der Trunkierung eines Gens für das Überleben der Motorneurone (SMN), das auf eine spinale Muskelatrophie oder eine amyotrophe Lateralsklerose hinweist, wobei die Methode die Schritte umfasst:
(a) Extrahieren von DNA aus einer Patientenprobe;
(b) Amplifizieren dieser DNA mit Primern gemäß Anspruch 11 oder Anspruch 12;
(c) Unterwerfen der amplifizierten DNA einem Einzelstrang-Konformationspolymorphismus, wobei die Analyse den Vergleich eines Musters von DNA-Fragmenten, die aus einer Patientenprobe erhalten wurden, mit einem Muster von DNA-Proben, die aus einer Kontrollprobe erhalten wurden, umfasst, um eine Trunkierung, eine Deletion oder eine Mutation im Gen des Patienten zu detektieren; und
(d) Detektieren des Vorliegens einer Trunkierung, einer Deletion oder einer Mutation im Patientengen in dem Gen für das Überleben der Motomeurone, das auf eine spinale Muskelatrophie oder eine amyotrophe Lateralsklerose hindeutet.

22. Methode gemäß Anspruch 21, wobei die Schritte (c) und (d) gegen einen Schritt eines Verdaus mit einem Bsrl-Enzym ausgetauscht werden.

23. In vitro Methode zur Detektion von spinaler Muskelatrophie, wobei die Methode die Schritte umfasst:
(a) Extrahieren von DNA aus einer Patientenprobe;
(b) Hybridisieren dieser DNA mit einer DNA-Sonde, die die gesamte oder einen Teil der DNA-Sequenz aus der Figur 3 umfasst, unter stringenten Bedingungen; und
(c) Detektieren der möglicherweise gebildeten Hybride, wobei wenn keine Hybridisierung auftritt, die Patientenprobe kein Gen für das Überleben der Motomeurone, das auf eine spinale Muskelatrophie hindeutet, enthält.

24. Methode gemäß Anspruch 23, wobei die Sonde radioaktiv markiert ist.

25. Monoklonaler Antikörper oder polyklonales Antiserum, der, bzw. das spezifisch an das SMN-Protein aus der Figur 1 oder das Protein aus der Figur 8 bindet.

26. In vitro-Methode zur Detektion der Abwesenheit oder der Unterbrechung des Gens für das Überleben der Motorneurone, das auf eine Arthrogryposis multiplex congenita (AMC) hinweist, wobei die Methode die Schritte umfasst:
(a) Extrahieren von DNA aus einer Patientenprobe;
(b) Amplifizieren der DNA mittels PCR unter Verwendung von unmarkierten Primern aus dem Exon 7 und Exon 8 des SMN-Gens;
(c) Unterwerfen dieser amplifizierten DNA einer Einzelstrang-Konformationspolymorphismus-Analyse, wobei die Analyse den Vergleich eines Musters von DNA-Fragmenten, die aus der Patientenprobe erhalten wurden, mit einem Muster von DNA-Fragmenten, die aus einer Kontrollprobe erhalten wurden, umfasst, um die Abwesenheit oder Unterbrechung in dem Gen des Patienten zu detektieren; und
(d) Detektieren der Abwesenheit oder der Unterbrechung eines Gens für das Überleben der Motorneurone, das auf eine Arthrogryposis multiplex congenita hinweist.

27. Transgene Maus, die das humane SMN-Protein der Figur 1 oder ein trunkiertes Protein der Figur 8 exprimiert.

28. Transgene Maus gemäß Anspruch 27, die durch homologe Rekombination erhalten ist.

29. Transgene Maus, die ein mutiertes SMN-Protein der Figur 1 exprimiert.

## Revendications

1. Gène humain isolé de survie des neurones moteurs ("survival motor neuron" - SMN) T-BCD541 qui présente la séquence d'ADNc qui est soulignée dans la figure 3A (nucléotides 34 à 915).

2. Gène de SMN isolé selon la revendication 1, qui comprend les séquences d'introns qui ont les séquences qui suivent :
- pour l'intron n°6 :
- pour l'intron n°7 :

3. Variante isolée du gène de SMN, laquelle variante est un gène C-BCD541 qui présente la séquence d'ADNc qui est soulignée dans la figure 2A (nucléotides 34 à 915).

4. Séquence de nucléotides isolée qui est la séquence d'ADNc du gène de SMN selon les revendications 1 ou 2, lequel ADNc présente la séquence qui est soulignée dans la figure 3A (nucléotides 34 à 915).

5. Séquence de codage du gène de SMN selon les revendications 1 ou 2, laquelle séquence comprend les nucléotides 34 à 915 de la séquence de la figure 3A.

6. Séquence de codage de la variante du gène de SMN selon la revendication 3, laquelle séquence comprend les nucléotides 34 à 915 de la séquence de la figure 2A.

7. Séquence d'introns du gène de SMN selon les revendications 1 ou 2, laquelle séquence présente l'une des séquences qui suivent :
- pour l'intron 6 :
- pour l'intron 7 :

8. Séquence d'ADN isolée qui code la protéine de survie des neurones moteurs (SMN) de la figure 1 ou de la figure 8.

9. Séquence de nucléotides isolée de la figure 11.

10. Oligonucléotide sélectionné parmi les séquences ci-dessous :

11. Ensemble d'oligonucléotides qui comprend un premier oligonucléotide qui présente au moins 9 nucléotides et qui est contenu dans une séquence selon l'une quelconque des revendications 5 à 7, pour autant que le premier oligonucléotide ne comprenne pas la séquence p(dT)₈dAdA ou la séquence p(dT)₈dA, et un deuxième oligonucléotide qui comprend au moins 9 nucléotides et qui est compris dans la séquence sélectionnée dans l'ensemble constitué des nucléotides 921 à 1 469 de la séquence de la figure 3A, l'intron 7 présentant la séquence ci-dessous : et la séquence de nucléotides de l'exon 7 de la figure 3A.

12. Ensemble d'amorces, qui comprend :
- deux amorces qui ont les séquences ci-dessous :
- deux amorces qui présentent les séquences ci-dessous :
- deux amorces qui présentent l'une des séquences ci-dessous :

13. Séquence de nucléotides antisens qui est une séquence complémentaire inverse d'une séquence selon l'une quelconque des revendications 5 à 7.

14. Protéine de survie des neurones moteurs (SMN) humaine isolée, qui comprend la séquence d'acides aminés de la figure 1.

15. Protéine selon la revendication 13, qui est tronquée et qui comprend la séquence de la figure 8.

16. Trousse pour la détection in vitro d'une troncature, d'une suppression ou d'une mutation d'un gène de survie des neurones moteurs (SMN) qui indique une atrophie musculaire de la colonne vertébrale ou une sclérose latérale amyotrophique, qui comprend :
- un ensemble d'oligonucléotides selon les revendications 10 ou 11,
- des réactifs destinés à une réaction d'amplification et
- une sonde qui détecte le produit amplifié, ladite sonde étant un fragment d'une séquence selon l'une quelconque des revendications 5 à 7, ledit fragment comprenant au moins 9 nucléotides.

17. Vecteur de clonage ou d'expression, **caractérisé en ce qu'**il comprend une séquence selon l'une quelconque des revendications 1 à 8.

18. Vecteur selon la revendication 17, **caractérisé en ce qu'**il est un poliovirus, un adénovirus ou un virus de l'herpès.

19. Vecteur selon la revendication 17, **caractérisé en ce qu'**il est un vecteur de rétrovirus.

20. Cellule hôte isolée, par exemple cellules de moelle osseuse, fibroblastes ou cellules épithéliales, **caractérisée en ce qu'**elle est transformée par un vecteur selon l'une quelconque des revendications 17 à 19.

21. Procédé de détection in vitro de la présence, l'absence ou la troncature d'un gène de survie des neurones moteurs (SMN) qui indiquent une atrophie musculaire de la colonne vertébrale ou une sclérose latérale amyotrophique, ledit procédé comprenant les étapes qui consistent à :
(a) extraire l'ADN d'un échantillon d'un patient,
(b) amplifier ledit ADN à l'aide d'amorces selon les revendications 11 ou 12,
(c) soumettre ledit ADN amplifié à un polymorphisme de conformation en simple brin, ladite analyse consistant à comparer un motif de fragments d'ADN obtenu sur un échantillon du patient à un motif d'échantillons d'ADN obtenu sur un échantillon témoin de manière à détecter une troncature, une suppression ou une mutation du gène du patient, et
(d) détecter la présence d'une troncature, d'une suppression ou d'une mutation du gène de survie des neurones moteurs du patient, ce qui indique une atrophie musculaire de la colonne vertébrale ou une sclérose latérale amyotrophique.

22. Procédé selon la revendication 21, dans lequel les étapes (c) et (d) sont remplacées par une étape de digestion avec une enzyme Bsrl.

23. Procédé in vitro pour détecter une atrophie musculaire de la colonne vertébrale, ledit procédé comprenant les étapes qui consistent à :
(a) extraire l'ADN d'un échantillon d'un patient,
(b) hybrider ledit ADN avec une sonde d'ADN qui comprend la totalité ou une partie de la séquence d'ADN de la figure 3 dans des conditions astringentes et
(c) détecter les hybrides éventuellement formés, et si aucune hybridation n'a lieu, l'échantillon de patient est dépourvu du gène de survie des neurones moteurs, ce qui indique une atrophie musculaire de la colonne vertébrale.

24. Procédé selon la revendication 23, dans lequel ladite sonde est marquée radioactivement.

25. Anticorps monoclonal ou antisérum monoclonal qui se lie en particulier à la protéine de SMN de la figure 1 ou à la protéine de la figure 8.

26. Procédé de détection in vitro de l'absence ou l'interruption d'un gène de survie des neurones moteurs qui indiquent une arthrogrypose congénitale multiple ("arthrogryposis multiplex congenita" - AMC), ledit procédé comprenant les étapes qui consistent à :
(a) extraire l'ADN d'un échantillon d'un patient,
(b) amplifier ledit ADN par l'intermédiaire d'une réaction en chaîne par polymérase ("polymerase chain reaction" - PCR) en utilisant des amorces non marquées de l'exon 7 et de l'exon 8 du gène de SMN,
(c) soumettre ledit ADN amplifié à un polymorphisme de conformation en simple brin, ladite analyse consistant à comparer un motif de fragments d'ADN obtenu sur un échantillon du patient à un motif d'échantillons d'ADN obtenu sur un échantillon témoin de manière à détecter l'absence ou l'interruption du gène du patient, et
(d) détecter l'absence ou l'interruption du gène de survie des neurones moteurs, qui indiquent une arthrogrypose congénitale multiple.

27. Souris transgénique qui exprime la protéine de SMN humaine de la figure 1 ou la protéine tronquée de la figure 8.

28. Souris transgénique selon la revendication 27, qui est obtenue par recombinaison homologue.

29. Souris transgénique qui exprime la protéine de SMN mutée de la figure 1.
